# EUROPEAN PATENT APPLICATION

(11) **EP 4 265 726 A1**
(43) Date of publication of application: **25.10.2023**
(21) Application number: 21906710.5
(22) Date of filing: 16.12.2021
(51) Int. Cl.: C12N 15/115, A61K 31/706, A61K 48/00, A61P 43/00, C07H 19/23, C12M 1/00, C12N 15/11, C12Q 1/68

(54) **DEOXYRIBONUCLEOSIDE OR DEOXYRIBONUCLEOTIDE HAVING NOVEL ARTIFICIAL BASE CAPABLE OF BEING USED FOR SCREENING FOR DNA APTAMER, AND NUCLEIC ACID CONTAINING SAME**

(30) Priority: 18.12.2020 JP 2020210488
(71) Applicant: TAGCyx Biotechnologies Inc., Tokyo 1530041 (JP)
(72) Inventor: MUTO, Susumu, Tokyo 153-0041 (JP); SOMEYA, Tatsuhiko, Tokyo 153-0041 (JP); HAYASE, Yoji, Tokyo 153-0041 (JP); JITSUZAKI, Daichi, Amagasaki-shi, Hyogo 660-0805 (JP)
(74) Representative: Schön, Christoph
(86) International application number: PCT/JP2021/046645
(87) International publication number: WO 2022/131350

(57) **Abstract**

An object of the present invention is to overcome problems in obtaining DNA aptamers in conventional methods, including the lack of diversification of interactions with target molecules, the increase in the cost for obtaining aptamers by post modification, and the limitation of target molecule species because of electric charges, and to provide a technique for obtaining highly active aptamers as inexpensively as possible. Provided is a nucleoside or nucleotide having, as a base, a structure of formula I. 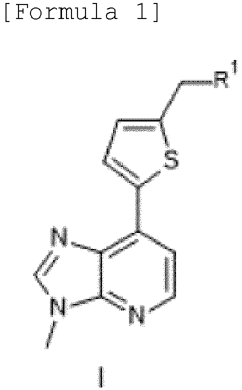 In formula I, R¹ is a substituted C₁-C₃ alkyl group, a substituted or unsubstituted C₄-C₉ alkyl group, a substituted or unsubstituted aryl group, a substituted or unsubstituted cycloalkyl group, a substituted or unsubstituted aralkyl group, or a substituted or unsubstituted heteroaryl group.

## Description

### [Technical Field]

The present invention relates to a deoxyribonucleoside or deoxyribonucleotide having a novel artificial base applicable to screening for DNA aptamers, and a nucleic acid containing it.

### [Background Art]

The DNA aptamer is a collective term for DNA molecular species that are single-stranded DNA having a chain length of 30 to 70 nucleotides and specifically interact with target molecules such as low-molecular-weight compounds and proteins by forming a thermodynamically stable specific three-dimensional structure via self-annealing. DNA aptamers, whose affinity with target molecules and selectivity of interactions with target molecules are comparable to those of antibodies, are also called synthetic antibodies, and considered promising as a modality for drug discovery of next generation.

Aptamer research has become more and more active year by year since the duration of the patent for the method Systematic Evolution of Ligands by EXponential enrichment (SELEX) (PTL 1) expired in 2011; however, no aptamer product, except Macugen ^{®}, has been successfully put on the market as a drug so far. The pharmacological characteristics of DNA aptamers are considered to be a main cause for that, and another putative cause is difficulty in obtaining aptamers that bind to target molecules because of the physical properties of DNA aptamers, in other words, the fact that the diversity of target molecules for which aptamers can be obtained is limited by the physical properties of DNA aptamers.

### [Citation List]

### [Patent Literature]

[PTL 1] US 5475096 A

### [Summary of Invention]

### [Technical Problem]

The followings are expected to be causes for the limitation of the diversity of target molecules for which DNA aptamers can be obtained.
(1) For DNA, nucleobases widely used as the constituent units are limited to four natural types (A, T, G, C). As compared with the fact that the number of existing natural amino acids is 20, the number of constituent units to form interactions is small for natural nucleobases, and accordingly the number of combinations for the formation of interaction is also small.
(2) For DNA, the base moieties of four natural nucleobases are used for direct interaction with target molecules such as proteins; however, the structures of the base moieties of natural type are poor in flexibility and not so lipophilic, and hence it cannot be said that the base moieties are proper as substructures expected to provide lipophilic interactions.
(3) Oligonucleotides including DNA have a negative charge at each phosphate-binding moiety. Thus, oligonucleotides are less likely to interact with negatively charged target molecules because of the occurrence of electrostatic repulsion, which limits the types of molecules that can be targeted by aptamers.

SomaLogic, Inc. in the US attempted to solve the limitation in creation of DNA aptamers by a method of introducing an artificial base having a flexible lipophilic substituent introduced therein in a manner by chemical synthesis after obtaining seed aptamers by the SELEX method (Post modification method). After trial and error, SomaLogic, Inc. has succeeded in obtaining aptamers that target 1000 or more proteins as target molecules (Larry Gold, et al., PLoS ONE, 2010, 5(11): e15004; Gupta S, et al., J. Biol. Chem. (2014) p8706-19).

However, there is currently no means to estimate the higher-order structure of oligonucleotide, and thus introduction of an artificial base having a lipophilic substituent introduced therein does not necessarily lead to enhanced activity. The post modification method has very poor efficiency and requires high cost because trial and error by synthesis of many derivatized aptamers is needed, and suffers from the limitation of activity to several tens of nM as Kd values, thus leaving room for improvement for an inexpensive method for obtaining aptamers. Further, DNA aptamers have a negative charge at each phosphate-binding moiety, hence being less likely to interact with negatively charged target molecules, as described above. Introduction of an artificial base having a positively charged substituent introduced therein, into a DNA aptamer is contemplated as a means to obtain DNA aptamers that bind to negatively charged target molecules or to improve for such DNA aptamers. For DNA aptamers obtained by the SELEX method, however, their phosphate-binding moieties are interacting with positively charged substructures of target molecules in most cases; accordingly, introduction of an artificial base having a positively charged substituent into an aptamer by post modification causes electrostatically generated repulsion, possibly being counterproductive. For this reason, introducing in advance an artificial base having a positively charged substituent introduced therein into a DNA library to be used in the SELEX method, rather than introducing a positively charged substituent with modification of base moieties after selection, is considered important and preferable for DNA aptamers to achieve sufficient interactions with negatively charged target molecules. In other words, it is expected to be difficult on the basis of the post modification method to efficiently obtain DNA aptamers for negatively charged target molecules.

On the other hand, Hirao et al. have developed a highly lipophilic artificial base pair (Ds-Px) that allows PCR with high fidelity ( JP 5424414 B2; Nucleic Acids Research, 2009 (37), e14) during their tenure at RIKEN, Japan, and succeeded in obtaining aptamers having very high affinities of several pM as Kd values for several proteins through selection of aptamers with introduction of only several Ds (7-(2-thienyl)-3H-imidazo[4,5-b]-pyridin-3-yl) units into a library to be used in SELEX (JP 6307675 B2; Michiko Kimoto, et al., Nature Biotechnology, 2013 (31), 453-457). A characteristic of this method is unnecessity of post modification, which allows DNA aptamers to be obtained inexpensively in a short period of time. However, the method has not succeeded in extending the diversity of target proteins for which DNA aptamers can be obtained. This is probably due to the fact that although Ds has higher lipophilicity than natural bases, Ds is poor in flexibility because of its structure and incapable of interacting with target molecules to a degree enough to overcome the influence of electric charges and result in binding.

The present invention has been made in view of such circumstances, and an object of the present invention is to overcome problems in obtaining DNA aptamers in the conventional methods described above, specifically, the lack of diversification of interactions with target molecules, the increase in the cost for obtaining aptamers by post modification, and the limitation of target molecule species because of electric charges, and to provide a technique for obtaining highly active aptamers as inexpensively as possible.

### [Solution to Problem]

To solve the problems, a novel artificial base applicable to screening for DNA aptamers and a nucleic acid containing it according to the present invention have the following configuration.

A first aspect of the present invention is a deoxyribonucleoside or deoxyribonucleotide having, as a base, a structure of formula I: 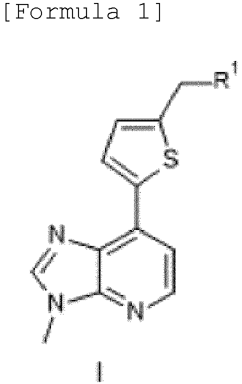 wherein R¹ is a substituted C₁-C₃ alkyl group, a substituted or unsubstituted C₄-C₉ alkyl group, a substituted or unsubstituted aryl group, a substituted or unsubstituted cycloalkyl group, a substituted or unsubstituted aralkyl group, or a substituted or unsubstituted heteroaryl group.

In the first aspect, in formula I, R¹ may be a phenyl group, a 4-pyridyl group, a naphthyl group, an isopropyl group, or a cyclopentyl group.

In the first aspect of the present invention, the deoxyribonucleotide may be a deoxyribonucleotide having triphosphate at the 5'-hydroxy group.

In the first aspect of the present invention, the deoxyribonucleoside may be a deoxyribonucleoside having a protecting group at the 5'-hydroxy group, and having a phosphoramidite group or an H-phosphonate group at the 3'-hydroxy group.

A second aspect of the present invention is a reagent for oligonucleotide synthesis, the reagent containing the deoxyribonucleoside or deoxyribonucleotide according to the first aspect.

A third aspect of the present invention is a medicament containing the nucleoside or nucleotide according to the first aspect.

A fourth aspect of the present invention is a synthesis method for the deoxyribonucleoside according to the first aspect, the method including a step of performing coupling reaction between a 2-bromothiophene derivative and 2-amino-3-nitro-4-chloropyridine or a 7-chloro-3H-imidazo[4,5-b]pyridine derivative.

In the fourth aspect, the coupling reaction is performed under conditions for Suzuki coupling reaction.

A fifth aspect of the present invention is an oligodeoxyribonucleotide incorporating a deoxyribonucleoside having the base according to the first aspect. The oligodeoxyribonucleotide may be a DNA aptamer.

A sixth aspect of the present invention is an oligonucleotide or a derivative thereof, the oligonucleotide containing the oligodeoxyribonucleotide according to the fifth aspect as a substructure.

A seventh aspect of the present invention is a modified product comprising the oligodeoxyribonucleotide according to the fifth aspect or the oligonucleotide or derivative thereof according to the sixth aspect bound to any independent molecular species.

An eighth aspect of the present invention is a medicament for a human or an animal, a raw material of the medicament, an additive for a health food, or a diagnostic reagent, wherein the medicament, the raw material, the additive, or the diagnostic reagent contains the oligodeoxyribonucleotide according to the fifth aspect, the oligonucleotide or derivative thereof according to the sixth aspect, or the modified product according to the seventh aspect.

A ninth aspect of the present invention is a medical article for the purpose of disease treatment, life support, management and control of symptoms, or management and control of systemic condition, the medical article containing the oligodeoxyribonucleotide according to the fifth aspect, the oligonucleotide or derivative thereof according to the sixth aspect, or the modified product according to the seventh aspect. The medical article may be a medical ingredient, a medical material, a medical device, or a medical product.

A tenth aspect of the present invention is a carrier for use in affinity column chromatography, the carrier containing the oligodeoxyribonucleotide according to the fifth aspect, the oligonucleotide or derivative thereof according to the sixth aspect, or the modified product according to the seventh aspect, a column including the carrier, or a substance separator or medical device including the carrier.

### [Advantageous Effects of Invention]

The nucleoside having an artificial base according to the present invention can be synthesized with a synthesis method including a step of performing coupling reaction between a 2-bromothiophene derivative and 2-amino-3-nitro-4-chloropyridine, without need of performing coupling reaction with use of a widely used tin-containing intermediate. This avoids discharge of wastes containing tin, and thus a synthesis method with reduced environmental loads is achieved. Further, scaled-up synthesis is achieved in synthesizing nucleosides.

Introduction of the deoxyribonucleoside having an artificial base according to the present invention into a DNA aptamer in advance allows the DNA aptamer to have enhanced ability to interact with the target molecule by virtue of a functional group possessed by the artificial base moiety. DNA incorporating the deoxyribonucleoside having an artificial base according to the present invention allows PCR with high fidelity, which enables introduction of a functional group possessed by the artificial base as early as in the stage of selection, and avoids the need of post modification. As a result, the number of steps for obtaining a DNA aptamer is reduced, which enables DNA aptamers to be obtained more inexpensively. Moreover, introducing a nucleoside having an artificial base containing a heterocycle having a positively charged functional group under physiological conditions into a single-stranded DNA library for SELEX allows achievement of interactions with negatively charged substructures, which have been considered difficult to generate.

An oligodeoxyribonucleotide incorporating a deoxyribonucleoside having the base according to the present invention allows achievement of proper interactions with a wider range of target proteins in a more flexible manner.

### [Brief Description of Drawings]

[Fig. 1] Fig. 1 is a diagram showing results of EMSA, which shows the affinities of DNA pools with a target protein, the DNA pools obtained through screening for VEGF165 by using a dDsbn form pool.
[Fig. 2] Fig. 2 is a diagram showing results of EMSA, which shows the binding affinity of a dDsbn form candidate sequence with VEGF165.
[Fig. 3] Fig. 3 is a diagram showing results of EMSA, which shows the affinities of DNA pools with a target protein, the DNA pools obtained through screening for sIL-6R by using a dDsbn form pool.
[Fig. 4] Fig. 4 is a diagram showing results of EMSA, which shows the binding affinity of a dDsbn form candidate sequence with sIL-6R.
[Fig. 5] Fig. 5 is a diagram showing results of EMSA, which shows the affinities of DNA pools with a target protein, the DNA pools obtained through screening for VEGF165 by using a dDscp form pool.
[Fig. 6] Fig. 6 is a diagram showing results of EMSA, which shows the binding affinity of a dDscp form candidate sequence with VEGF165.
[Fig. 7] Fig. 7 is a diagram showing results of EMSA, which shows the affinities of DNA pools with a target protein, the DNA pools obtained through screening for sIL-6R by using a dDscp form pool.
[Fig. 8] Fig. 8 is a diagram showing results of EMSA, which shows the binding affinity of a dDscp form candidate sequence with sIL-6R.

### [Description of Embodiments]

In view of the aforementioned problems inherent in the conventional techniques, the present inventors thought that providing an artificial base that is applicable to the SELEX method, that is, that allows incorporated DNA to be subjected to PCR with high fidelity and to interact with target molecules in a more flexible manner can be a solution to the problems, and diligently studied, specifically, for developing an artificial base satisfying the following conditions:
(1) being applicable to chemical synthesis as a unit of DNA or oligonucleotide;
(2) being amplifiable through PCR;
(3) having a substructure capable of properly interacting with target molecules in a flexible manner; and
(4) having a positively charged substructure under conditions that allow interactions with target molecules.

The present inventors modified an artificial base Ds (7-(2-thienyl)-3H-imidazo[4,5-b]-pyridin-3-yl), which has been known to be amplifiable through PCR and applicable even to the SELEX method, by introducing thereto a lipophilic substituent or a substituent having a cationic substructure, and thereby eventually completed the invention according to the present embodiment.

The introduction of a lipophilic substituent is expected to provide, for example, the following effects.
- Improvement in affinity through the formation of more effective hydrophobic interaction with target molecules.
- Promotion of the formation of a complex with a target molecule in water through reduction in water solubility due to the enhanced lipophilicity of the entire molecule.

The introduction of a substituent having a cationic substructure is expected to provide, for example, the following effects.
- Proper interactions are expected even for negatively charged target molecules, which have been previously thought to be difficult for DNA aptamers to bind to, and aptamers can be obtained with higher probability.
- Aptamers can be obtained with higher probability even for sugar chains, for which it has been previously thought to be difficult to obtain aptamers.

Hereinafter, embodiments of a deoxyribonucleoside or deoxyribonucleotide having the artificial base according to the present embodiment, and an oligodeoxyribonucleotide containing it will be described.

### 1. Nucleoside and nucleotide having artificial base

The artificial base according to the present embodiment can be synthesized with a method including a step of performing coupling reaction with 2-amino-3-nitro-4-chloropyridine. Coupling reaction without use of any widely used tin-containing intermediate can be achieved by performing that coupling reaction under conditions for Suzuki coupling reaction. This avoids discharge of wastes containing tin, and thus a synthesis method with reduced environmental loads is achieved as compared with conventional methods using a tin-containing intermediate. In addition, scaled-up synthesis is achieved.

Herein, "artificial base" refers to an artificially synthesized nucleobase analog having characteristics similar to those of a base moiety of a natural nucleoside present in nature. The artificial base according to the present embodiment has a complementary artificial base that together forms a base pair in a selective and specific manner. In the artificial base pair, not only hydrogen bonds used for base pairing between natural bases, but also physical bonds based on the structures or shapes of the artificial bases, stacking interactions such as π-π interactions, and electrostatic bonds due to positively/negatively charged sites can be formed. Herein, "Ds" refers to 7-(2-thienyl)-3H-imidazo[4,5-b]-pyridin-3-yl, one of artificial bases.

Herein, "nucleoside" refers to a compound in which a base moiety and a sugar are bonded via a glycoside bond. Herein, the term "nucleotide" refers to a compound in which the 5'- or 3'-hydroxy group of a nucleoside is bonding to phosphoric acid to form phosphate.

Herein, "lipophilic substituent" refers to a substituent composed only of carbon, hydrogen, and halogen atoms. Herein, "cationic substituent" refers to a substituent that contains in the configuration a nitrogen atom, which is protonated and positively charged in the pH range of 4 to 8 to form a cation. Herein, "derivative of artificial base" refers to a base analog formed by substituting a part of an artificial base with the aforementioned lipophilic substituent or cationic substituent, or with another functional group.

The nucleoside having an artificial base according to the present embodiment can be used even as a medicament. From the similarity of the structure of the artificial base according to the present embodiment to adenosine, for example, it is contemplated to use the artificial base according to the present embodiment as an adenosine receptor modulator (Dilip K Tosh, et al., J. Med. Chem., 2012 (55), 4847-4860).

### 2. Nucleoside phosphoramidite and nucleoside triphosphate having artificial base

A 3'-phosphoramidite of the nucleoside having an artificial base according to the present embodiment with the 5'-hydroxy group protected with a suitable protecting group can be used as a constituent of DNA or oligonucleotide for chemical synthesis of DNA by a solid-phase synthesis method under the same conditions as for natural bases. Specifically, a 5'-hydroxy group-protected 3'-phosphoramidite of the nucleoside having an artificial base according to the present embodiment can be combined with natural or modified nucleoside phosphoramidite to synthesize DNA having a desired chain length with a widely used chemical synthesis apparatus for DNA.

The phosphoramidite moiety may be widely used 2-cyanoethyl-N,N'-diisopropyl phosphoramidite, the 2-cyanoethyl group may be another phosphate protecting group, and the N,N'-diisopropyl phosphoramidite may be another appropriate amino group. A cyclic amidite in which N and O are bonded together is also permitted.

A 5'-triphosphate of the nucleoside having an artificial base according to the present embodiment can be synthesized with a widely used chemical synthesis method.

### 3. Nucleic acid containing nucleoside having artificial base

The nucleotide having an artificial base according to the present embodiment can form a base pair with the complementary artificial base, Px, and thus functions in PCR. Specifically, addition of a 5'-triphosphate of a nucleoside having Px together with a 5'-triphosphate of the nucleoside having an artificial base according to the present embodiment to PCR solution in advance allows the artificial base according to the present embodiment and Px to form a base pair, and such double-stranded DNA is amplified through PCR. Accordingly, the nucleotide having an artificial base according to the present embodiment can be used for screening for aptamers by the SELEX method, for which PCR is needed. The number of artificial bases introduced in DNA synthesis by PCR amplification is one to five in one DNA molecule in suitable cases, and preferably three or less. If a plurality of artificial bases is contained in one DNA molecule, the artificial bases contained in one DNA molecule may be the same, and a different artificial base that forms a base pair may be contained. Further, a base pair may be formed in one single strand to form a secondary structure or a tertiary structure.

"Aptamer" generally refers to an oligonucleotide or peptide that binds to a specific molecule and has a middle molecular size (10 to 100 nucleotides), or a derivative or modified product thereof. An oligonucleotide or DNA that contains the artificial base according to the present embodiment and binds to a specific molecule is categorized as a DNA aptamer.

A DNA aptamer can be used as it is, and can be used, as necessary, even as an adduct formed by adding to the 5' or 3' end an oligonucleotide having another function such as protecting and/or stabilizing the primary structure and secondary or tertiary structure of the DNA aptamer against enzymatic decomposition and decomposition caused by physical stimulation such as light and heat, a PEGylated (PEG: polyethylene glycol) or glycosylated modified product, a modified product such as a complex with a drug and an antibody-aptamer complex, a homo-polyvalent aptamer such as divalent one, or a hetero-polyvalent aptamer such as bispecific one.

DNA aptamers produced by introducing the artificial base according to the present embodiment are applicable as medicaments for humans or animals or raw materials thereof, medical ingredients, tools for drug delivery, tools or modules for separation/purification of specific substances or cells such as affinity column chromatography, sensors for detecting specific substances, additives for health foods, and diagnostic reagents. When a DNA aptamer is used for affinity column chromatography, a substance can be separated as a form selectively binding to the DNA aptamer by subjecting a sample to column chromatography with a carrier to which the DNA aptamer has been bound. Specific examples of application of affinity column chromatography for a medical ingredient or a medical device include apheresis.

Herein, "nucleic acid", "oligonucleotide", or "nucleic acid molecule" refers to a biopolymer in which multiple nucleotides are linked together via phosphodiester bonds. Herein, "DNA" refers to a biopolymer in which deoxyribonucleotides are linked together via phosphodiester bonds. Optionally, some of phosphodiester moieties binding nucleotides may be each modified phosphate such as phosphorothioate. Single-stranded or double-stranded DNA falls within the category of "nucleic acid", "oligonucleotide", or "nucleic acid molecule" herein. Optionally, "nucleic acid", "oligonucleotide", or "nucleic acid molecule" herein may form a triplex or quadruplex, and, further optionally, may be a chimeric molecule of DNA and RNA. Optionally, position 2' of any deoxyribose or ribose forming a nucleoside in "nucleic acid", "oligonucleotide", or "nucleic acid molecule" herein may be modified with fluorine, a methoxy group, or the like.

### [Examples]

### [Example 1]

### Synthesis of 7-(5-benzylthiophen-2-yl)-3-(2-deoxy-1-β-D-ribofuranosyl)-3H-imidazo[4,5-b]pyridine (compound 1)

### <Step 1: Synthesis of 2-bromo-5-benzylthiophene>

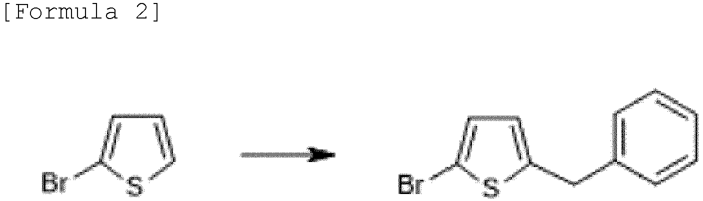

Under an argon atmosphere, 2-bromothiophene (50.0 g, 307 mmol) was dissolved in tetrahydrofuran (700 ml), and the resultant was cooled in a dry ice/acetone bath (internal temperature: -60°C). Tetramethylethylenediamine (55.5 ml, 368 mmol) was added thereto, 1.1 M n-hexane/tetrahydrofuran solution of lithium diisopropylamide (355 ml, 368 mmol) was added dropwise thereto while the internal temperature was kept at -55°C or lower, and the resultant was stirred for 1 hour with keeping the conditions. Benzyl bromide (43.6 mL, 368 mol) was added to the reaction solution, the temperature was increased to room temperature, and the reaction solution was stirred for 20 hours. After confirming the disappearance of the raw materials, saturated ammonium chloride (1 L) was added to the reaction solution, which was then subjected to extraction twice with heptane/ethyl acetate = 1/1 solution. The organic layers were combined, washed with brine, then subjected to liquid separation, and dried over anhydrous sodium sulfate. The anhydrous sodium sulfate was removed through filtration, and the filtrate was concentrated under reduced pressure. The residue from concentration was purified by silica gel chromatography (eluent: heptane) to afford 2-bromo-5-benzylthiophene (29.1 g) as a white solid.
¹H-NMR (CDCl₃, 400 MHz) δ (ppm): 4.06 (s, 2H), 6.54-6.55 (m, 1H), 6.85 (d, J = 3.7 Hz, 1H), 7.21-7.25 (m, 3H), 7.28-7.33 (m, 2H).

### <Step 2: Synthesis of pinacol 5-benzylthiophen-2-ylboronate>

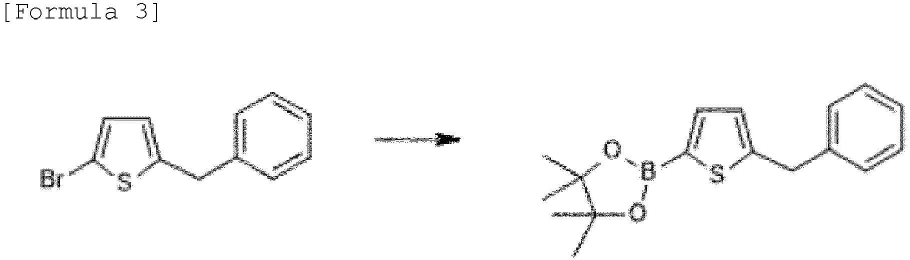

In 1,4-dioxane (200 mL), 2-bromo-5-benzylthiophene (20.2 g, 80 mmol) was dissolved, bis(pinacolato)diboron (40.5 g, 160 mmol), a dichloromethane adduct of [1,1'-bis(diphenylphosphino)ferrocene]palladium(II) dichloride (4.89 g, 5.98 mmol), and potassium acetate (23.5 g, 239 mmol) were added thereto under an argon atmosphere, and the resultant was stirred at 90°C for 2 hours. After confirming the disappearance of the raw materials, a mixed solution of water and heptane/ethyl acetate = 1/1 solution was added to the reaction solution, which was stirred and then subjected to liquid separation. The aqueous layer was further subjected to extraction once with heptane/ethyl acetate = 1/1 solution. The organic layers were combined, then washed with brine, dried over anhydrous sodium sulfate, and filtered. The filtrate was concentrated under reduced pressure, and the residue was purified by silica gel chromatography (neutral silica gel, eluent: heptane/ethyl acetate = 1/0 → 9/1) to afford pinacol 5-benzylthiophen-2-ylboronate (12.3 g) as a yellow solid.
¹H-NMR (CDCl₃, 400 MHz) δ (ppm): 1.31 (s, 12H), 4.18 (s, 2H), 6.87-6.89 (m, 1H), 7.19-7.31 (m, 5H), 7.47 (2.3 Hz, 1H).

### <Step 3: Synthesis of 2-amino-3-nitro-4-(5-benzylthiophen-2-yl)pyridine>

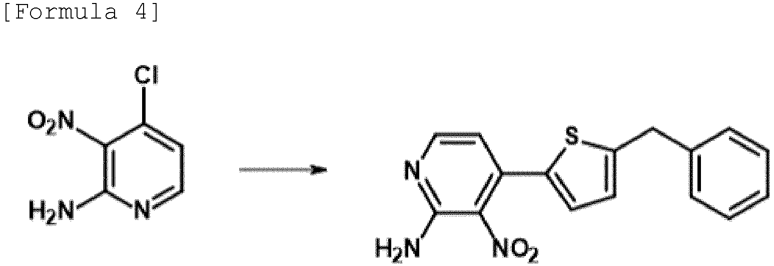

In 1,4-dioxane/water = 10/1 (242 mL), pinacol 5-benzylthiophen-2-ylboronate (12.3 g) and 2-amino-3-nitro-4-chloropyridine (5.69 g, 32.8 mmol) were dissolved, the resultant was then degassed, cesium carbonate (21.4 g, 65.6 mmol) and tetrakis(triphenylphosphine)palladium(0) (1.89 g, 1.64 mmol) were added thereto under an argon atmosphere, and the resultant was stirred at 90°C for 2 hours 30 minutes. The disappearance of the raw materials was confirmed, and a mixed solution of water and ethyl acetate was added to the reaction solution, which was then stirred and subjected to liquid separation. The aqueous layer was further subjected to extraction once with ethyl acetate, and the organic layers were combined, then dried over anhydrous sodium sulfate, filtered, and concentrated under reduced pressure. The residue from concentration was purified by silica gel chromatography (neutral silica gel, eluent: heptane/ethyl acetate = 1/0 → 7/3) to afford 2-amino-3-nitro-4-(5-benzylthiophen-2-yl)pyridine (9.20 g) as an orange solid.
¹H-NMR (CDCl₃, 400 MHz) δ (ppm): 4.16 (s, 2H), 5.63 (bs, 2H), 6.72 (d, J = 5.0 Hz, 1H), 6.76-6.78 (m, 1H), 6.97 (d, J = 3.7 Hz) 7.26-7.35 (m, 5H), 8.12 (d, J = 5.5 Hz, 2H).

### <Step 4: Synthesis of 2,3-diamino-4-(5-benzylthiophen-2-yl)pyridine>

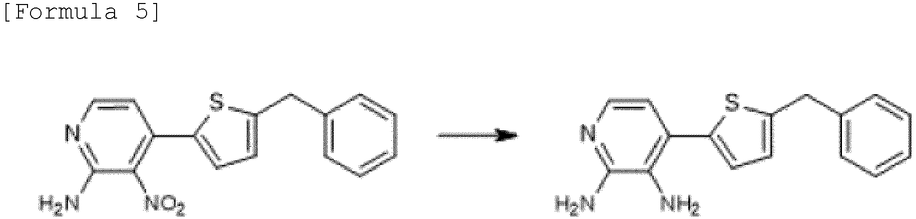

In tetrahydrofuran (100 mL), 2-amino-3-nitro-4-(5-benzylthiophen-2-yl)pyridine (9.80 g) was dissolved. Palladium carbon (NE-chemcat K type, 50% wet, 1.3 g) was added thereto, purging with hydrogen was performed three times, and stirring was then performed at room temperature for 24 hours with installation of a hydrogen balloon.

The disappearance of the raw materials was confirmed, purging with Ar was performed and filtration through a Celite was then performed, and the residue was thoroughly washed with methanol. The filtrate was concentrated under reduced pressure to afford 2,3-diamino-4-(5-benzylthiophen-2-yl)pyridine (10.1 g) as a blackish brown oily substance.
¹H-NMR (CDCl₃, 400 MHz) δ (ppm): 3.79 (bs, 2H), 4.16 (s, 2H), 4.42 (bs, 2H), 6.68 (d, J = 5.0 Hz, 1H), 6.81-6.83 (m, 1H), 7.05 (d, J = 3.7 Hz, 1H), 7.23-7.35 (m, 5H), 7.57 (d, J = 5.5 Hz, 1H).

### <Step 5: Synthesis of 7-(5-benzylthiophen-2-yl)-3H-imidazo[4,5-b]pyridine>

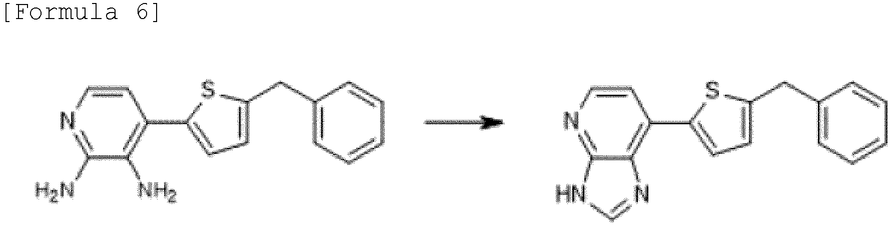

In triethyl orthoformate (157 mL, 945 mmol), 2,3-diamino-4-(5-benzylthiophen-2-yl)pyridine (10.1 g) was dissolved, 35% hydrochloric acid (6.01 mL, 69.3 mmol) was slowly added thereto, and the resultant was stirred at room temperature for 4 hours. After confirming the disappearance of the raw materials, the reaction solution was filtered, and the residual matter was washed with ethyl acetate. The residual matter was added to a mixed solution of saturated aqueous solution of sodium hydrogen carbonate and chloroform/methanol = 9/1 solution to dissolve therein, and the resultant was subjected to liquid separation. The aqueous layer was further subjected to extraction once with chloroform/methanol = 9/1 solution. The organic layers were combined, dried over anhydrous sodium sulfate, and filtered, and the filtrate was concentrated under reduced pressure. The residue from concentration was suspended in and washed with acetonitrile twice to afford 7-(5-benzylthiophen-2-yl)-3H-imidazo[4,5-b]pyridine (6.12 g) as a pale brown solid.
¹H-NMR (DMSO-d₆, 400 MHz) δ (ppm): 4.23 (s, 2H), 7.04 (d, J = 4.1 Hz, 1H), 7.23-7.26 (m, 1H), 7.32-7.37 (m, 4H), 7.45 (d, J = 5.0 Hz, 1H), 8.09 (d, J = 3.7 Hz, 1H), 8.27 (d, J = 5.0 Hz, 1H), 8.43 (s, 1H).

### <Step 6: Synthesis of 7-(5-benzylthiophen-2-yl)-3-[3,5-di-O-(p-toluoyl)-2-deoxy-1-β-D-ribofuranosyl]-3H-imidazo[4,5-b]pyridine>

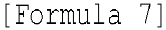

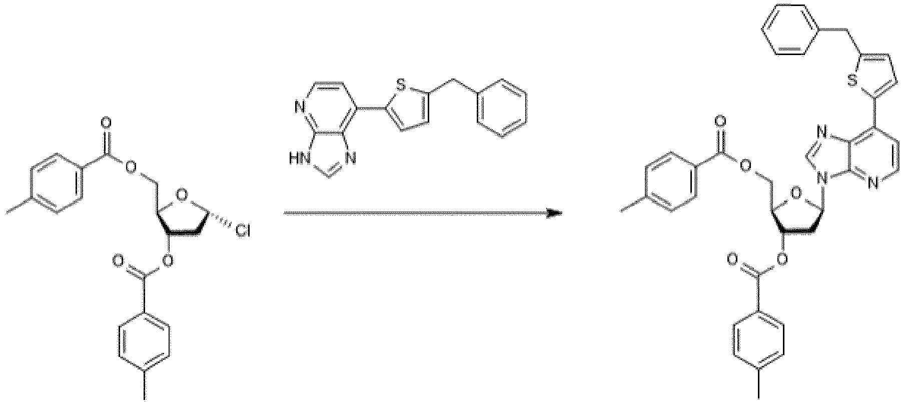

Under an argon atmosphere, 7-(5-benzylthiophen-2-yl)-3H-imidazo[4,5-b]pyridine (5.50 g, 18.9 mmol) was dissolved in acetonitrile (88 mL), sodium hydride (60 wt%, 0.75 g, 18.9 mmol) was added thereto in an ice bath, the temperature was then increased to room temperature, and stirring was performed for 1 hour 30 minutes. After stirring, 2-deoxy-3,5-di-O-(p-toluoyl)-α-D-erythrohentofuranosyl chloride (6.12 g, 15.7 mmol) was added to the reaction solution in an ice bath, and the reaction solution was stirred at room temperature for 2 hours 30 minutes. The disappearance of the raw materials was confirmed, a mixed solution of saturated aqueous solution of ammonium chloride and ethyl acetate was added thereto, and the resultant was stirred and then subjected to liquid separation. The aqueous layer was further subjected to extraction once with ethyl acetate, and the organic layers were combined, then dried over anhydrous sodium sulfate, filtered, and concentrated under reduced pressure. The residue from concentration was purified by silica gel chromatography (neutral silica gel, eluent: heptane/ethyl acetate = 1/0 → 1/1) to afford 7-(5-benzylthiophen-2-yl)-3-[3,5-di-O-(p-toluoyl)-2-deoxy-1-β-D-ribofuranosyl]-3H-imidazo[4,5-b]pyridine (4.61 g) as a yellow amorphous solid.
¹H-NMR (CDCl₃, 400 MHz) δ (ppm) : 2.38 (s, 3H), 2.44 (s, 3H), 2.85 (ddd, J = 14.2 Hz, 5.5 Hz, 1.8 Hz, 1H), 3.16-3.19 (m, 1H), 4.21 (s, 1H), 4.64-4.77 (m, 3H), 5.83 (d, J = 5.9 Hz, 1H), 6.68 (dd, J = 8.5 Hz, 5.7 Hz, 1H), 6.89 (d, J = 3.7 Hz, 1H), 7.19-7.34 (m, 9H), 7.38 (d, J = 5.0 Hz, 1H), 7.90 (d, J = 8.2 Hz, 2H), 7.98-7.99 (m, 3H), 8.25 (s, 1H), 8.29 (d, J = 5.0 Hz, 1H).

### <Step 7: Synthesis of 7-(5-benzylthiophen-2-yl)-3-(2-deoxy-1-β-D-ribofuranosyl)-3H-imidazo[4,5-b]pyridine (compound 1)>

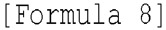

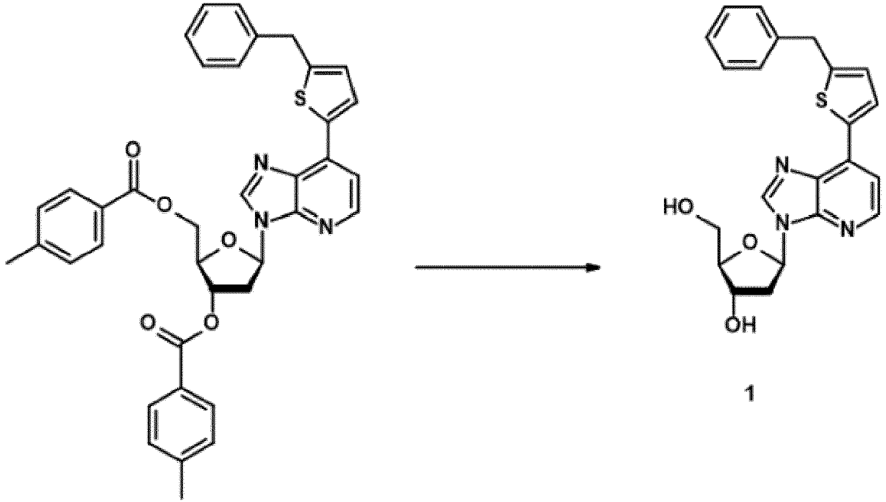

To ammonia (7 M methanol solution ,410 mL, 2.86 mol), 7-(5-benzylthiophen-2-yl)-3-[3,5-di-O-(p-toluoyl)-2-deoxy-1-β-D-ribofuranosyl]-3H-imidazo[4,5-b]pyridine (4.61 g, 7.16 mmol) was added, and the resultant was stirred at room temperature for 48 hours. The disappearance of the raw materials was confirmed, and the reaction solution was concentrated under reduced pressure. The residue from concentration was purified by silica gel chromatography (eluent: heptane/ethyl acetate = 1/1 → 0/1 → chloroform/methanol = 1/0 → 9/1) to afford compound 1 (2.74 g) as a white amorphous solid.
¹H-NMR (DMSO-d₆, 400 MHz) δ (ppm): 2.34 (ddd, J = 13.2 Hz, 6.0 Hz, 3.0 Hz, 1H), 2.75-2.82 (m, 1H), 3.52-3.57 (m, 1H), 3.63-3.66 (m, 1H), 3.91 (dd, J = 7.3 Hz, 4.6 Hz, 1H), 4.23 (s, 2H), 4.44-4.46 (m, 1H), 5.12 (t, J = 5.7 Hz), 5.34 (d, J = 3.7 Hz), 6.52 (t, J = 7.3 Hz, 1H), 7.05 (d, J = 3.7 Hz, 1H), 7.24-7.26 (m, 1H), 7.32-7.37 (m, 4H), 7.54 (d, J = 5.5 Hz), 8.12 (d, J = 3.7 Hz), 8.30 (d, J = 5.0 Hz, 1H), 8.70 (s, 1H).

Although a deoxynucleoside (compound 1) containing a base having a benzyl group at position 5 of the thiophene of Ds was synthesized in Example 1 above, a deoxynucleoside having another functional group at position 5 of the thiophene of Ds can be synthesized with the same method as in synthesis of compound 1.

### [Example 2]

### Synthesis of 7-(5-benzylthiophen-2-yl)-3-[2-deoxy-5-O-(4,4'-dimethoxytrityl)-1-β-D-ribofuranosyl]-3H-imidazo[4,5-b]pyridine 2-cyanoethyl-N,N'-diisopropylphosphoramidite (compound 2)

### <Step 1: Synthesis of 7-(5-benzylthiophen-2-yl)-3-[2-deoxy-5-O-(4,4'-dimethoxytrityl)-1-β-D-ribofuranosyl]-3H-imidazo[4,5-b]pyridine>

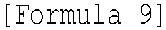

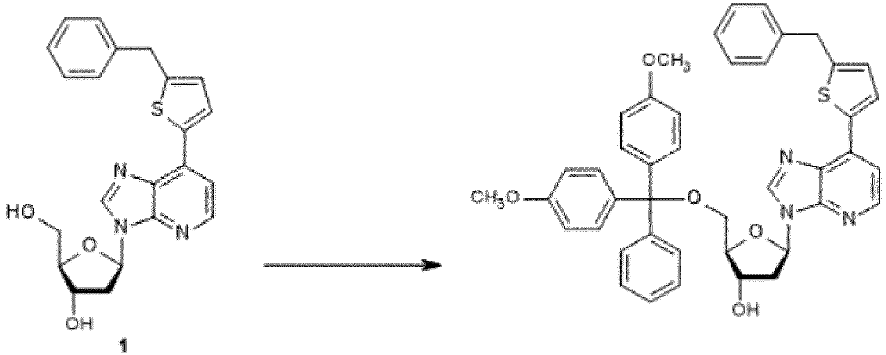

After being azeotroped with pyridine (three times), compound 1 (0.5 g, 1.23 mmol) produced in Example 1 was dissolved in pyridine (5 mL). DMTrCl (0.46 g, 1.35 mmol) was added thereto under ice-cooling, and the resultant was stirred at room temperature overnight. Methanol (1 mL) was added thereto under ice-cooling to quench the reaction, the solvent was distilled off under reduced pressure, and the residue was purified by silica gel column chromatography (mixed solution of dichloromethane and methanol with 1% triethylamine, methanol concentration: 0 → 3%) to afford 7-(5-benzylthiophen-2-yl)-3-[2-deoxy-5-O-(4,4'-dimethoxytrityl)-1-β-D-ribofuranosyl]-3H-imidazo[4,5-b]pyridine (0.64 g).
Mass: ESI(-) 708.28

### <Step 2: Synthesis of 7-(5-benzylthiophen-2-yl)-3-[2-deoxy-5-O-(4,4'-dimethoxytrityl)-1-β-D-ribofuranosyl]-3H-imidazo[4,5-b]pyridine 2-cyanoethyl-N,N'-diisopropylphosphoramidite (compound 2)>

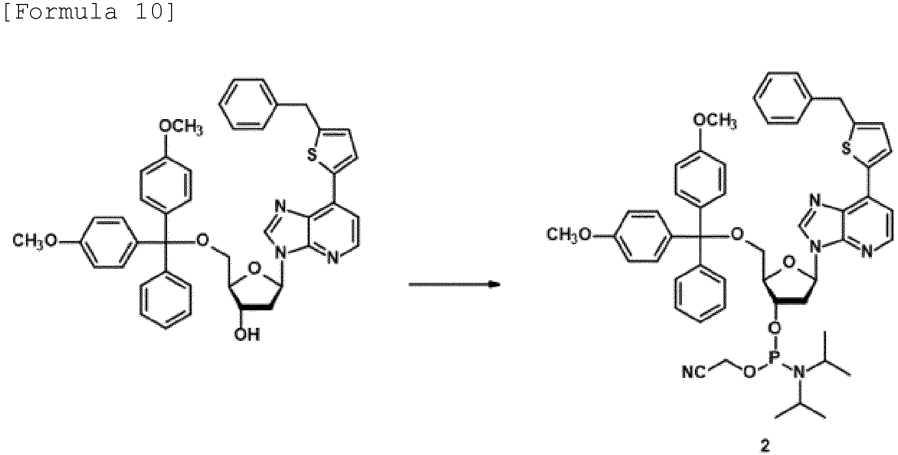

After being azeotroped with acetonitrile (three times), 7-(5-benzylthiophen-2-yl)-3-[2-deoxy-5-O-(4,4'-dimethoxytrityl)-1-β-D-ribofuranosyl]-3H-imidazo[4,5-b]pyridine (0.60 g, 0.85 mmol) was dissolved in acetonitrile (10 mL). Under ice-cooling, 1H-tetrazole (82 mg, 1.02 mmol) and 2-cyanoethyl-tetraisopropylphosphorodiamidite (0.32 mL, 1.02 mmol) were added thereto, and the resultant was stirred at room temperature for 2 hours, then diluted with 30 mL of ethyl acetate, and washed with NaHCO₃ water and brine. The organic layer was dried over anhydrous sodium sulfate and then filtered, and the solvent was distilled off under reduced pressure. The residue was purified by silica gel column chromatography (mixed solution of hexane and ethyl acetate with 1% triethylamine, ethyl acetate concentration: 5% → 30% → 50%) to afford 7-(5-benzylthiophen-2-yl)-3-[2-deoxy-5-O-(4,4'-dimethoxytrityl)-1-β-D-ribofuranosyl]-3H-imidazo[4,5-b]pyridine 2-cyanoethyl-N,N'-diisopropylphosphoramidite (0.66 g).

A 3'-phosphoramidite of a deoxynucleoside having a benzyl group at position 5 of the thiophene of Ds was synthesized in Example 2 above. Alternatively, a 3'-phosphoramidite of a deoxynucleoside having another functional group at position 5 of the thiophene of Ds can be synthesized with the same method.

### [Example 3]

### Synthesis of 7-(5-benzylthiophen-2-yl)-3-(2-deoxy-1-β-D-ribofuranosyl)-3H-imidazo[4,5-b]pyridine 5'-triphosphate

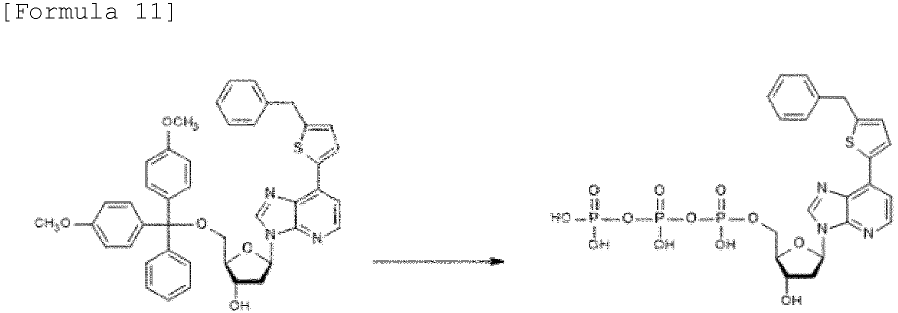

In pyridine (9 mL), 7-(5-benzylthiophen-2-yl)-3-[2-deoxy-5-O-(4,4'-dimethoxytrityl)-1-β-D-ribofuranosyl]-3H-imidazo[4,5-b]pyridine (0.63 g, 0.89 mmol) obtained in step 1 of Example 2 was dissolved. Acetic anhydride (0.17 mL, 1.78 mmol) was added thereto, and the resultant was stirred at room temperature overnight. The solvent was distilled off under reduced pressure, and the resultant was then diluted with ethyl acetate and washed with sodium bicarbonate water and brine. The organic layer was dried over anhydrous sodium sulfate and then filtered, and the solvent was distilled off under reduced pressure. Toluene was added to the residue for azeotroping (three times), and the resultant was dissolved in dichloromethane (90 mL). Dichloroacetic acid (0.9 mL) was added thereto under ice-cooling, and the resultant was stirred for 25 minutes. The solvent was distilled off under reduced pressure, and the resultant was then diluted with dichloromethane and washed with sodium bicarbonate water and brine. The organic layer was dried over anhydrous sodium sulfate and filtered, and the solvent was then distilled off under reduced pressure. The residue was purified by silica gel column chromatography (dichloromethane-methanol, methanol concentration: 0% → 3%) to afford a 3'-O-acetyl form of 7-(5-benzylthiophen-2-yl)-3-[2-deoxy-5-O-(4,4'-dimethoxytrityl)-1-β-D-ribofuranosyl]-3H-imidazo[4,5-b]pyridine as an intermediate. Pyridine was added to this compound (90 mg, 0.2 mmol) for azeotroping (three times), and the resultant was dissolved in pyridine (0.2 mL) and 1,4-dioxane (0.8 mL). Thereto, 1 M 1,4-dioxane solution of 2-chloro-4-H-1,3,2-benzodioxaphosphorin-4-one (0.3 mL) was added, and the resultant was stirred at room temperature for 15 minutes. Thereto, n-butylamine (0.2 mL) and 0.5 M DMF solution of tributylammonium pyrophosphate(0.8 mL) were added, the resultant was stirred at room temperature for 15 minutes, 4 mL of 1% iodide/pyridine-water (49:1) was then added thereto, and the resultant was stirred at room temperature for 15 minutes. Thereto, 5% sodium hydrogen sulfite (0.3 mL) was added, and the resultant was stirred at room temperature for 2 minutes and then concentrated under reduced pressure to a volume of approximately 1 mL. Water (10 mL) was added to the residue, the resultant was stirred at room temperature for 30 minutes, 28% ammonia water (40 mL) was then added thereto, and the resultant was stirred at room temperature for 3.5 hours and concentrated under reduced pressure to a volume of approximately 5 mL. The residue was roughly purified by DEAE column chromatography (buffer A: 50 mM triethylammonium acetate solution, buffer B: 2 M triethylammonium acetate solution, linear gradient). The resultant was further purified by reverse-phase HPLC (buffer A: 5% acetonitrile, 50 mM triethylammonium acetate solution, buffer B: acetonitrile, buffer B/20% → 85%, 13 minutes). The resulting fraction was concentrated, azeotroped with water multiple times, and then freeze-dried. The residue was redissolved in water, giving 33.7 mM aqueous solution (0.5 mL), to afford the target product 7-(5-benzylthiophen-2-yl)-3-(2-deoxy-1-β-D-ribofuranosyl)-3H-imidazo[4,5-b]pyridine 5'-triphosphate.
Mass: ESI(-) 646.21

A 5'-triphosphate of a deoxynucleoside containing a base having a benzyl group at position 5 of the thiophene of Ds was synthesized in Example 3 above. A 5'-triphosphate of a deoxynucleoside having another functional group at position 5 of the thiophene of Ds can be synthesized with the same method.

### [Example 4]

### Synthesis of 7-(5-cyclopentylmethylthiophen-2-yl)-3-(2-deoxy-1-β-D-ribofuranosyl)-3H-imidazo[4,5-b]pyridine (compound 3)

### <Step 1: Synthesis of 2-bromo-5-(cyclopentylidenemethyl)thiophene>

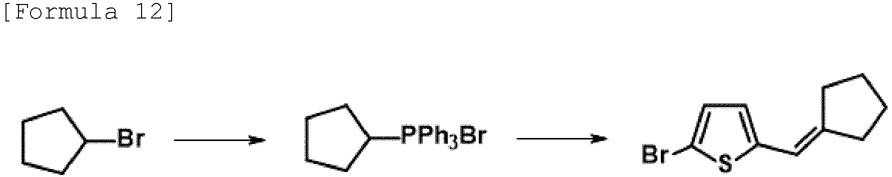

Under an argon atmosphere, triphenylphosphine (123.57 g, 471 mmol) was added to bromocyclopentane (78.0 g, 523 mmol), and the resultant was heated to 135 to 140°C and stirred for 4.5 hours with keeping the conditions. After cooling to 40°C, toluene (100 mL) was added thereto, the resultant was filtered to collect a solid, and the solid from filtration was washed with toluene and dried under reduced pressure to afford cyclopentyltriphenylphosphonium bromide (135.9 g, 82 wt%, yield: 57.5%).

Under an argon atmosphere, 2-bromo-4-formylthiophene (40.0 g, 209 mmol) was dissolved in THF (1L), and a solution of cyclopentyltriphenylphosphonium bromide (126 g, 251 mmol) in THF (1L) was added thereto. The reaction suspension was heated to 60°C, and NaH (1.26 g, 26.2 mmol, 60 wt% product) was added 25 times every 15 minutes (total amount added: 31.5 g, 655 mmol). After the completion of adding all NaH followed by stirring for 15 minutes, the reaction suspension was cooled to 30°C and poured into iced water (4 L), the resultant was subjected to extraction twice with ethyl acetate (2 L), and the organic layer was washed with 20% saline and then concentrated under reduced pressure. Heptane (500 mL) was added to the residue from concentration, the residue was suspended (ultrasonication), and the suspended matter was collected by filtration. The product from filtration was washed with heptane, and the filtrate was concentrated under reduced pressure. The residue from concentration was purified by column chromatography (silica gel, 1 kg, heptane) to afford 2-bromo-5-(cyclopentylidenemethyl)thiophene (34.2 g, 67.3%).
¹H-NMR (CDCl₃, 400 MHz) δ (ppm): 1.65-1.72 (m, 2H), 1.80-1.87 (m, 2H), 2.40-2.46 (m, 4H), 6.47-6.48 (m, 1H), 6.61 (d, J = 3.7 Hz, 1H), 6.93 (d, J = 4.1 Hz, 1H)

### <Step 2: Synthesis of 2-[5-(cyclopentylidenemethyl)-2-thienyl]-4,4,5,5-tetramethyl-1,3,2-dioxaborolane>

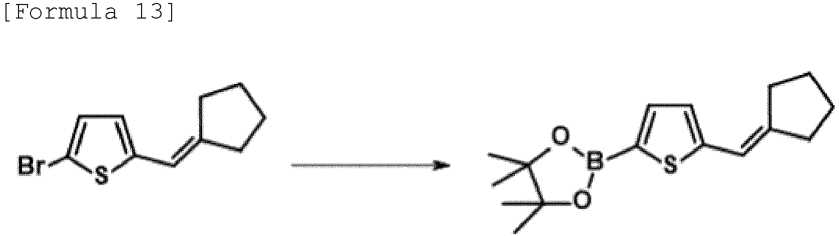

In dioxane (287 mL), 2-bromo-5-(cyclopentylidenemethyl)thiophene (29.0 g, 119 mmol) was dissolved. Bis(pinacolato)diboron (60.6 g, 239 mmol) and potassium acetate (35.1 g, 358 mmol) were added thereto, Pd(dppf)Cl₂·DCM (0.970 g, 1.19 mmol) was further added thereto under an argon atmosphere, and the reaction mixture was heated to 90°C and stirred for 2 hours 40 minutes with keeping the conditions. Pd(dppf)Cl₂·DCM (0.1 g, 0.122 mmol) was additionally added thereto, and the resultant was stirred under heating for 1 hour 10 minutes. Thereafter, the reaction mixture was cooled and poured into a mixed solvent of water (500 mL)-heptane (300 mL)-ethyl acetate (300 mL), and insoluble matters were removed by filtration. The organic layer was subjected to liquid separation, and the aqueous layer was again subjected to extraction with heptane (200 mL)-ethyl acetate (200 mL). The organic layer was washed with brine (400 mL), dried over sodium sulfate, and concentrated under reduced pressure to afford a crude product (82.9 g). Heptane (300 mL) was added thereto, and the resultant was again concentrated under reduced pressure. Heptane (80 ml) was added to the residue from concentration to suspend the residue, and the suspended matter was collected by filtration, and washed with heptane. The filtrate was purified by column chromatography (silica gel: 300 g, elution with heptane) to afford 2-[5-(cyclopentylidenemethyl)-2-thienyl]-4,4,5,5-tetramethyl-1,3,2-dioxaborolane (60.5 g, 100%, containing bis(pinacolato)diboron as a residue).
¹H-NMR (CDCl₃, 400 MHz) δ (ppm): 1.34 (s, 12H), 1.65-1.72 (m, 2H), 1.80-1.86 (m, 2H), 2.46-2.55 (m, 4H), 6.64 (m, 1H), 6.94 (d, J = 3.7 Hz, 1H), 7.53 (d, J = 3.7 Hz, 1H)

### <Step 3: Synthesis of 4-[5-(cyclopentylidenemethyl)-2-thienyl]-3-nitro-pyridine-2-amine>

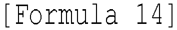

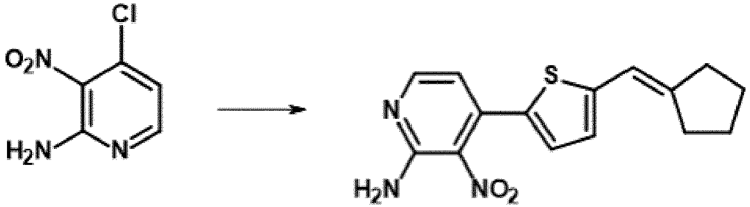

In dioxane (173 mL), 2-amino-4-chloro-3-nitropyridine (9.60 g, 55.3 mmol) and 2-[5-(cyclopentylidenemethyl)-2-thienyl]-4,4,5,5-tetramethyl-1,3,2-dioxaborolane (55.7 g, 192 mmol) were dissolved. Water (17.3 mL) and cesium carbonate (36.0 g, 111 mmol) were added thereto, and the inside of the vessel was purged with argon. Pd(PPh₃)₄ (3.20 g, 2.77 mmol) was added thereto, and the inside of the vessel was again purged with argon. The reaction mixture was heated to approximately 90°C and stirred for 1 hour with keeping the conditions. The temperature of the reaction mixture was returned to room temperature, the reaction mixture was poured into water (500 mL)-ethyl acetate (500 mL), and insoluble matters were removed by filtration through a Celite. The organic layer was subjected to liquid separation, and the aqueous layer was again subjected to extraction with ethyl acetate (500 mL). The organic layer was washed with brine (300 mL) (here, removal of insoluble matters was performed as well), dried over sodium sulfate, and concentrated under reduced pressure to afford a crude product (44.3 g). The crude product was purified by column chromatography (silica gel: 500 g, ethyl acetate: heptane = 10:90 → 15:85 → 25:75 → 30:70) to afford 4-[5-(cyclopentylidenemethyl)-2-thienyl]-3-nitro-pyridine-2-amine (11.5 g, impure). The resulting 4-[5-(cyclopentylidenemethyl)-2-thienyl]-3-nitro-pyridine-2-amine (impure) was halved, and each was again purified by column chromatography (silica gel: 200 g, chloroform:ethyl acetate = 100:0 → 90:10) to afford 4-[5-(cyclopentylidenemethyl)-2-thienyl]-3-nitro-pyridine-2-amine (5.07 g, 30.4%, a mixture with 2-amino-4-chloro-3-nitropyridine).

### <Step 4: Synthesis of 4-[5-(cyclopentylidenemethyl)-thienyl]pyridine-2,3-diamine>

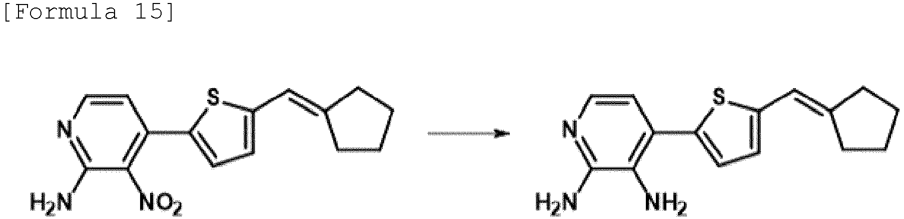

In THF (43 mL), 4-[5-(cyclopentylidenemethyl)-2-thienyl]-3-nitro-pyridine-2-amine (4.3 g, 14.27 mmol) was dissolved, 10%-Pd/C (50% wet, 0.56 g, 2.63 mmol) was added thereto under an argon atmosphere, the inside of the vessel was further purged with hydrogen (24 → 22°C), and stirring was performed under the hydrogen atmosphere at room temperature for 20 hours (balloon, internal temperature: 25 to 26°C). After the inside of the vessel was purged with argon, the reaction mixture was filtered through a Celite, which was then washed with THF. The filtrate was concentrated under reduced pressure to afford a crude product (4.83 g). The crude product was purified by column chromatography (NH silica gel: 100 g, CHCl₃:heptane = 50:50 → 85:15), and a portion containing impurities was again purified by column chromatography (NH silica gel: 60 g, CHCl₃:heptane = 30:70 → 65:35) to afford 4-[5-(cyclopentylidenemethyl)-thienyl]pyridine-2,3-diamine (1.81 g, 46.8%).
¹H-NMR (CDCl₃, 400 MHz) δ (ppm): 1.68-1.75 (m, 2H), 1.82-1.89 (m, 2H), 2.48-2.55 (m, 4H), 3.77 (bs, 2H), 4.25 (bs, 2H), 6.59 (t, J = 2.3 Hz, 1H), 6.77 (d, J = 5.5 Hz, 1H), 6.91 (d, J = 3.7 Hz, 1H), 7.15 (d, J = 3.7 Hz, 1H), 7.65 (d, J = 5.0 Hz, 1H)

### <Step 5: Synthesis of 4-[5-(cyclopentylmethyl)-thienyl]pyridine-2,3-diamine

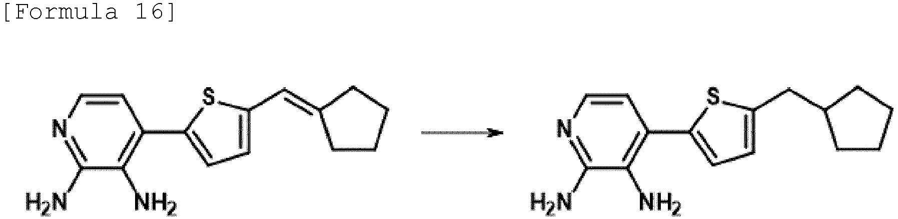

In an autoclave reactor, 4-[5-(cyclopentylidenemethyl)-thienyl]pyridine-2,3-diamine (1.81 g, 6.67 mmol) was dissolved in THF (20.1 mL), and the inside of the vessel was purged with argon. Thereto, 10%-Pd/C (50% wet, 0.26 g, 1.228 mmol) was added, and the inside of the vessel was pressurized with hydrogen at 0.6 MPa, and stirring was performed at room temperature for 6.5 hours (with depressurization to 0.38 MPa). Again, pressurization was performed with hydrogen at 0.6 MPa, and stirring was performed at room temperature for 64 hours (with depressurization to 0.23 MPa). The pressure in the vessel was returned to normal pressure and purging with argon was performed, and the reaction mixture was filtered through a Celite. The Celite was washed with THF, and the filtrate was concentrated under reduced pressure. Methanol and toluene were added thereto, and the resultant was again concentrated under reduced pressure to afford 4-[5-(cyclopentylmethyl)-thienyl]pyridine-2,3-diamine (1.82 g, 100%).

### <Step 6: Synthesis of 7-(5-cyclopentylmethylthiophen-2-yl)-3-(2-deoxy-1-β-D-ribofuranosyl)-3H-imidazo[4,5-b]pyridine (compound 3)>

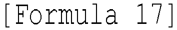

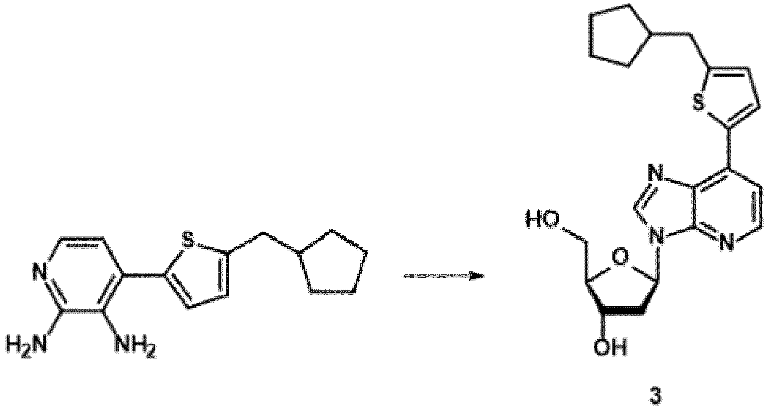

By using 4-[5-(cyclopentylmethyl)-thienyl]pyridine-2,3-diamine (1.8 g) as a raw material, 555 mg of 7-(5-cyclopentylmethylthiophen-2-yl)-3-(2-deoxy-1-β-D-ribofuranosyl)-3H-imidazo[4,5-b]pyridine (compound 3) was obtained with the same method as in steps 4 to 7 of Example 1. ¹H-NMR (CDCl₃, 400 MHz) δ (ppm): 1.22-1.31 (m, 2H), 1.52-1.60 (m, 2H), 1.62-1.70 (m, 2H), 1.77-1.87 (m, 2H), 2.15-2.26 (m, 2H), 2.31 (dd, J = 5.7, 13.5 Hz, 1H), 2.88 (d, J = 7.3 Hz, 2H), 3.20-3.27 (m, 1H), 3.81 (t, J = 12.3 Hz, 1H), 4.00 (d, J = 12.8, 1H), 4.26 (s, 1H), 4.83 (d, J = 4.1 Hz, 1H), 6.43 (dd, J = 5.5, 9.6 Hz, 1H), 6.80 (d, J = 11.4 Hz, 1H), 6.89 (d, J = 3.7 Hz, 1H), 7.44 (d, J = 5.5 Hz, 1H), 8.00 (d, J = 3.7 Hz, 1H), 8.12 (s, 1H), 8.25 (d, J = 5.5 Hz, 1H)

### [Example 5]

### Synthesis of phosphoramidite of compound 3

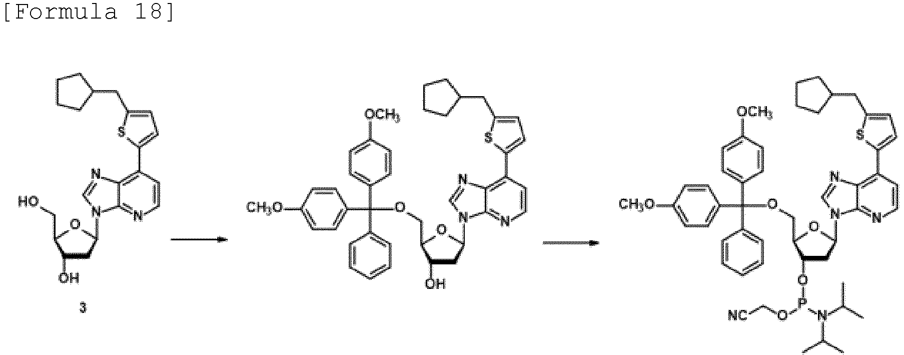

By using 7-(5-cyclopentylmethylthiophen-2-yl)-3-(2-deoxy-1-β-D-ribofuranosyl)-3H-imidazo[4,5-b]pyridine (compound 3), which was obtained in Example 4, as a raw material, 7-(5-cyclopentylmethylthiophen-2-yl)-3-[2-deoxy-5-O-(4,4'-dimethoxytrityl)-1-β-D-ribofuranosyl]-3H-imidazo[4,5-b]pyridine 2-cyanoethyl-N,N'-diisopropylphosphoramidite (0.66 g) was obtained through production with the same method as in Example 2.

### [Example 6]

### Synthesis of triphosphate of compound 3

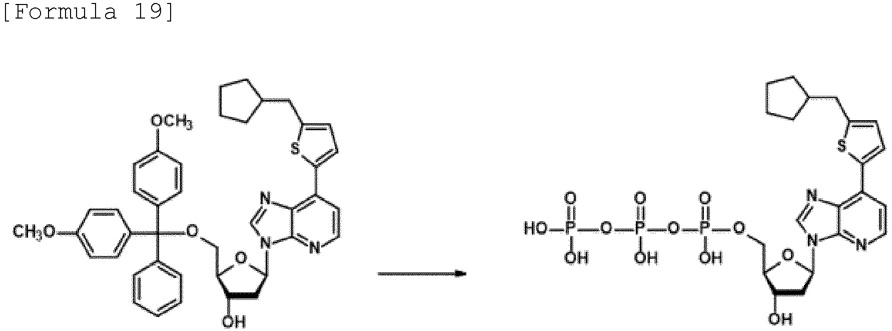

By using 7-(5-cyclopentylmethylthiophen-2-yl)-3-[2-deoxy-5-O-(4,4'-dimethoxytrityl)-1-β-D-ribofuranosyl]-3H-imidazo[4,5-b]pyridine, which is an intermediate obtained in Example 5, as a raw material, 7-(5-cyclopentylmethylthiophen-2-yl)-3-[2-deoxy-5-O-(4,4'-dimethoxytrityl)-1-β-D-ribofuranosyl]-3H-imidazo[4,5-b]pyridine 5'-triphosphate was obtained through production with the same method as in Example 3.
Mass: ESI(-) 638.10

### [Example 7]

### Synthesis of 7-(2-naphthylmethylthiophen-2-yl)-3-(2-deoxy-1-β-D-ribofuranosyl)-3H-imidazo[4,5-b]pyridine (compound 4)

### <Step 1: Synthesis of 2-bromo-5-(2-naphthylmethyl)thiophene>

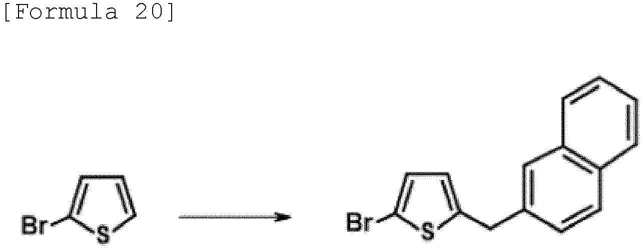

In a 1L-four-necked flask under an argon atmosphere, 2-bromothiophene (7.35 g, 45.1 mmol) was dissolved in tetrahydrofuran (103 mL), tetramethylethylenediamine (8.16 mL, 54.1 mmol, 1.2 eq.) was added thereto, and the resultant was then cooled in a dry ice/acetone bath (internal temperature: - 60°C or lower). Subsequently, lithium diisopropylamide (1.08 M n-hexane/tetrahydrofuran solution, 50.0 mL, 54.1 mmol, 1.2 eq.) was added dropwise thereto over 20 minutes, and the resultant was stirred at the same temperature for 30 minutes. A solution of 2-bromomethyl naphthalene (12.0 g, 54.1 mmol, 1.2 eq.) in tetrahydrofuran (20 mL) was added dropwise thereto, the resultant was then taken out of the dry ice/acetone bath and stirred for 19 hours after the temperature was increased to room temperature.

After confirming the disappearance of the raw materials, saturated aqueous solution of ammonium chloride (100 mL) and ethyl acetate (100 mL) were added thereto for liquid separation, and the aqueous layer was again subjected to extraction with ethyl acetate (100 mL). The organic layers were combined, washed with brine (100 mL), then dried over anhydrous sodium sulfate, and filtered. The filtrate was concentrated under reduced pressure to afford a brown solid (21.0 g). Purification was performed by silica gel column chromatography (FUJI SILYSIA CHEMICAL LTD. SI50 SIZE 200 × 4; n-heptane), and the targeted fraction was concentrated under reduced pressure to afford 2-bromo-5-(2-naphthylmethyl)thiophene (4.27 g, yield: 31%) as a white solid.
¹H-NMR (CDCl₃, 400 MHz) δ (ppm): 4.23 (s, 2H), 6.59-6.61 (m, 1H), 6.88 (d, J = 3.6 Hz, 1H), 7.35 (dd, J = 1.8, 8.2 Hz, 1H), 7.43-7.49 (m, 2H), 7.67 (s, 1H), 7.78-7.83 (m, 3H)

### <Step 2: Synthesis of 4,4,5,5-tetramethyl-2-[5-(2-naphthylmethyl)-2-thienyl]-1,3,2-dioxaborolane>

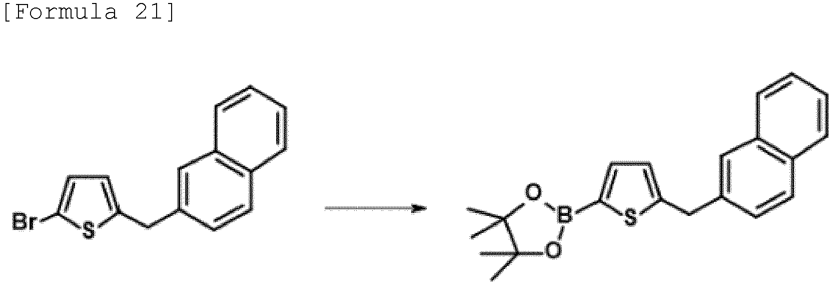

In a 100 mL-four-necked flask under an argon atmosphere, 2-bromo-5-(2-naphthylmethyl)thiophene (4.27 g, 14.1 mmol) and bispinacolatodiboron (3.75 g, 14.8 mmol, 1.05 eq.) were dissolved in dioxane (35.3 mL). After adding potassium acetate (4.15 g, 42.2 mmol, 3.0 eq.), the atmosphere in the flask was changed to an argon atmosphere, PdCl₂(dppf) (860 mg, 1.06 mmol, 0.075 eq.) was added, and the resultant was then stirred at an internal temperature of 90°C for 24 hours. After confirming the disappearance of the raw materials, the reaction solution was cooled to room temperature, and filtered through a Celite. Water (50 mL) and ethyl acetate (100 mL) were added to the filtrate, which was then subjected to liquid separation. The organic layer was washed with brine (200 mL), then dried over anhydrous sodium sulfate, and filtered. The filtrate was concentrated under reduced pressure, the residue was purified by silica gel column chromatography (eluent: n-heptane:ethyl acetate = 100:0 → 90:10), and the targeted fraction was concentrated under reduced pressure to afford 4,4,5,5-tetramethyl-2-[5-(2-naphthylmethyl)-2-thienyl]-1,3,2-dioxaborolane (2.61 g, yield: 53%).
¹H-NMR (CDCl₃, 400 MHz) δ (ppm): 1.31 (s, 12H), 4.34 (s, 2H), 6.92-6.93 (m, 1H), 7.37 (dd, J = 1.8, 8.6 Hz, 1H), 7.42-7.49 (m, 3H), 7.69 (s, 1H), 7.76-7.81 (m, 3H)

### <Step 3: Synthesis of [(2R)-5-(7-chloroimidazo[4,5-b]pyridin-3-yl)-3-(4-methylbenzoyl)oxy-tetrahydrofuran-2-yl]methyl 4-methylbenzoate>

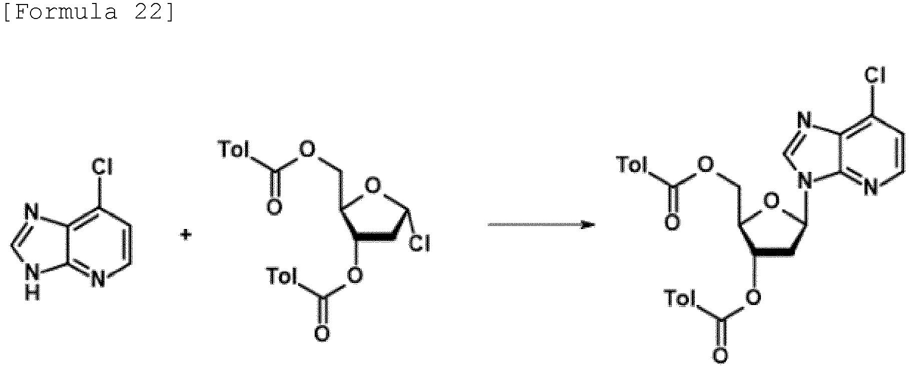

A 1L-four-necked flask under an argon atmosphere was charged with 7-chloro-3H-imidazo[4,5-b]pyridine (2.00 g, 13.2 mmol), NaH (60%, 630 mg, 15.6 mmol, 1.20 eq.), and acetonitrile (429 mL) under ice-cooling, and the resultant was stirred. After adding [(2R,5R)-5-chloro-3-(4-methylbenzoyl)oxy-tetrahydrofuran-2-yl]methyl 4-methylbenzoate (6.85 g, 17.6 mmol, 1.35 eq.), the resultant was stirred at room temperature for 1 hour (colorless transparent solution). After confirming the disappearance of the raw materials, saturated aqueous solution of ammonium chloride (100 mL) and ethyl acetate (100 mL) were added to the reaction solution, which was then subjected to liquid separation. Thereafter, extraction was performed twice with ethyl acetate (100 mL). The organic layer was washed with brine (200 mL), then dried over anhydrous sodium sulfate, and filtered. The filtrate was concentrated under reduced pressure to afford a yellow solid (10.0 g). Purification was performed by silica gel column chromatography (eluent: n-heptane:ethyl acetate = 100:0 , 50:50), the targeted fraction was concentrated under reduced pressure, the resulting residue was again purified by silica gel column chromatography (eluent: chloroform:methanol = 100:0 → 20:1), and the targeted fraction was concentrated under reduced pressure to afford [(2R)-5-(7-chloroimidazo[4,5-b]pyridin-3-yl)-3-(4-methylbenzoyl)oxy-tetrahydrofuran-2-yl]methyl 4-methylbenzoate (3.31 g, yield: 50%) as a white solid.
¹H-NMR (CDCl₃, 400 MHz) δ (ppm): 2.41 (s, 3H), 2.45 (s, 3H), 2.85 (ddd, J = 2.2, 5.6, 14.1 Hz, 1H), 3.08-3.19 (m, 1H), 4.63-4.92 (m, 3H), 5.83 (dt, J = 1.8, 6.0 Hz, 1H), 6.65 (dd, J = 5.8, 8.0 Hz, 1H), 7.18-7.30 (m, 5H), 7.90 (d, J = 8.0 Hz, 2H), 7.98 (d, J = 8.0 Hz, 2H), 8.26-8.27 (m, 2H)

### <Step 4: Synthesis of [(2R)-3-(4-methylbenzoyl)oxy-5-[7-[5-(2-naphthylmethyl)-2-thienyl]imidazolo[4,5b]pyridin-3-yl]tetrahydrofuran-2-yl]methyl 4-methylbenzoate>

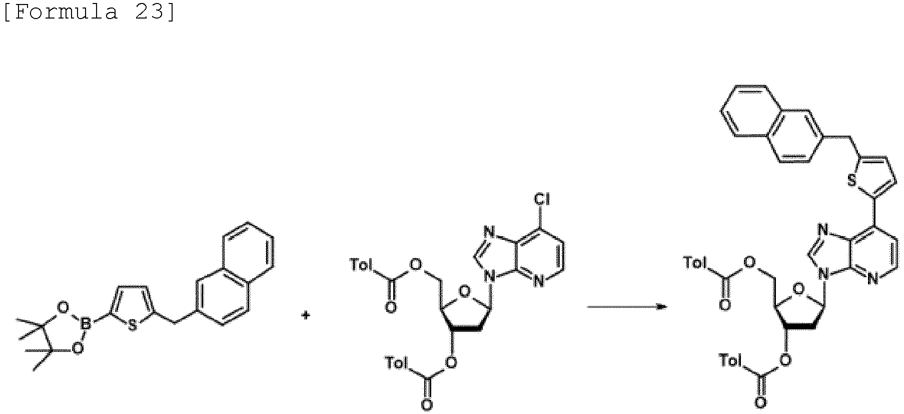

In a 200mL-four-necked flask under an argon atmosphere, 4,4,5,5-tetramethyl-2-[5-(2-naphthylmethyl)-2-thienyl]-1,3,2-dioxaborolane (830 mg, 1.58 mmol) and [(2R)-5-(7-chloroimidazo[4,5-b]pyridin-3-yl)-3-(4-methylbenzoyl)oxy-tetrahydrofuran-2-yl]methyl 4-methylbenzoate (831 mg, 2.37 mmol, 1.5 eq.) were dissolved in dioxane (8.00 mL), and the resultant was stirred. After adding aqueous solution of cesium carbonate (2.50 g, 3.16 mmol, 3 eq.)/water (4.00 mL), the inside of the reaction vessel was purged with argon, Xphos Pd G2 (0.05 g, 0.0320 mmol, 0.05 eq.) was added, and the resultant was then stirred at 90°C for 1 hour. After confirming the disappearance of the raw materials, water (40 mL) and ethyl acetate (40 mL) were added to the reaction solution, which was then subjected to liquid separation. Thereafter, extraction was performed twice with ethyl acetate (40 mL). The organic layer was washed with brine (200 mL), then dried over anhydrous sodium sulfate, and filtered. The filtrate was concentrated under reduced pressure to afford a yellow solid (2.01 g). Purification was performed by silica gel column chromatography (eluent: n-heptane:ethyl acetate = 85:15 , 70:30), and the targeted fraction was concentrated under reduced pressure to afford [(2R)-3-(4-methylbenzoyl)oxy-5-[7-[5-(2-naphthylmethyl)-2-thienyl]imidazolo[4,5b]pyridin-3-yl]tetrahydrofuran-2-yl]methyl 4-methylbenzoate (789 mg, yield: 76%) as a white solid.
¹H-NMR (CDCl₃, 400 MHz) δ (ppm): 2.38 (s, 3H), 2.44 (s, 3H), 2.85 (ddd, J = 2.2, 5.6, 14.1 Hz, 1H), 3.14-3.21 (m, 1H), 4.38 (s, 2H), 4.66-4.76 (m, 3H), 5.83 (dt, J = 1.8, 6.0 Hz, 1H), 6.68 (dd, J = 5.8, 8.2 Hz, 1H), 6.94 (d, J = 4.0 Hz, 1H), 7.20 (d, J = 8.4 Hz, 2H), 7.28 (d, J = 8.4 Hz, 2H), 7.38-7.49 (m, 4H), 7.74-7.83 (m, 4H), 7.91 (d, J = 8.4 Hz, 2H), 7.98 (d, J = 8.0 Hz, 2H), 8.01 (d, J = 4.0 Hz, 1H), 8.25 (s, 1H), 8.29 (d, J = 5.2 Hz, 1H)

<Step 5: Synthesis of 7-(2-naphthylmethylthiophen-2-yl)-3-(2-deoxy-1-β-D-ribofuranosyl)-3H-imidazo[4,5-b]pyridine (compound 4)>

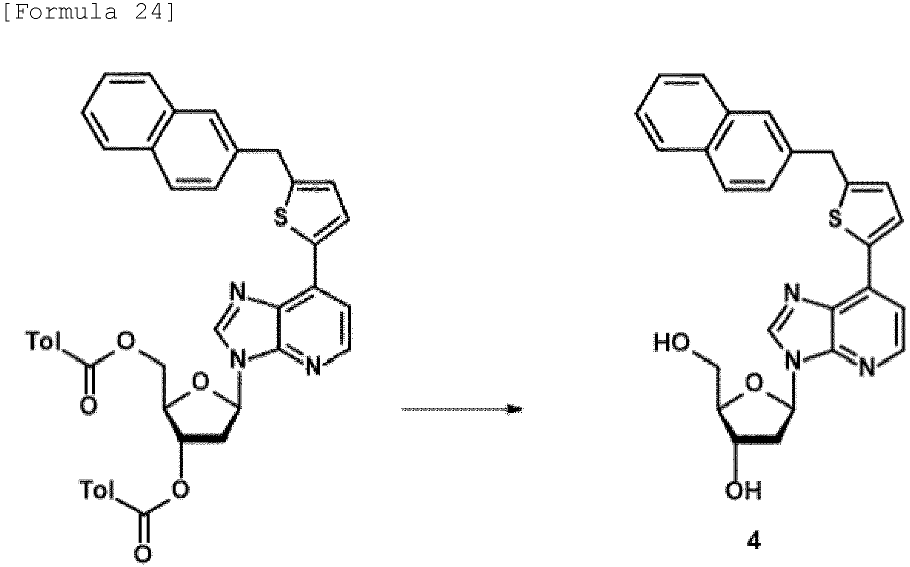

From [(2R)-3-(4-methylbenzoyl)oxy-5-[7-[5-(2-naphthylmethyl)-2-thienyl]imidazolo[4,5b]pyridin-3-yl]tetrahydrofuran-2-yl]methyl 4-methylbenzoate (0.959 g) produced in step 4, 620 mg (99%) of 7-(2-naphthylmethylthiophen-2-yl)-3-(2-deoxy-1-β-D-ribofuranosyl)-3H-imidazo[4,5-b]pyridine (compound 4) was obtained with the same method as in step 7 of Example 1. ¹H-NMR (CDCl₃, 400 MHz) δ (ppm): 1.89 (s, 1H), 2.31 (dd, J = 5.2, 14.1 Hz, 1H), 3.20-3.27 (m, 1H), 3.78-3.84 (m, 1H), 3.98-4.01 (m, 1H), 4.25 (s, 1H), 4.38 (s, 2H), 4.83 (t, J = 4.0 Hz, 1H), 6.42 (dd, J = 5.6, 10.0 Hz, 1H), 6.69-6.72 (m, 1H), 6.94-6.96 (m, 1H), 7.40-7.49 (m, 4H), 7.75 (s, 1H), 7.79-7.83 (m, 3H), 8.03 (d, J = 3.6 Hz, 1H), 8.10 (s, 1H), 8.24 (d, J = 5.2 Hz, 1H)

### [Example 8]

### Synthesis of DNA using 3'-amidite of nucleoside having artificial base

In common solid-phase synthesis methods, nucleoside 3'-amidite containing an artificial base according to the present embodiment, an example of which is compound 2, is applicable to synthesis of oligonucleotides under conditions comparable to those for nucleoside 3'-amidite having a natural base. Compound 2 was applied to a solid-phase synthesis method, and the resulting DNA strand was purified by polyacrylamide gel electrophoresis. The mass of the DNA strand purified was analyzed from its MS spectrum to find that the molecular weight was 31850.2 (theoretical value: 31852.9); thus, it was confirmed that DNA of interest containing 7-(5-benzyl-2-thienyl)-3H-imidazo[4,5-b]-pyridin-3-yl (hereinafter, referred to as Dsbn) was successfully obtained.

### [Example 9]

### Application of nucleoside 5'-triphosphate having artificial base to PCR

To confirm that the nucleotide having the artificial base 7-(5-benzyl-2-thienyl)-3H-imidazo[4,5-b]-pyridin-3-yl as its base moiety (hereinafter, referred to as dDsbn) is incorporated in a DNA strand by DNA polymerase and amplified, DNA fragments obtained through PCR were analyzed by LC/MS.

The single-stranded DNA containing dDsbn, which was prepared in Example 8, was used as a template for the following PCR.

PCR was carried out with AccuPrime Pfx DNA polymerase (manufactured by Thermo Fisher Scientific). The composition of PCR solution was as follows: lx AccuPrime Pfx Reaction Mix (containing 0.3 mM dNTP, 1.0 mM MgSO₄), 0.5 mM MgSO₄, 0.1 mM dNTP, 0.5 µM Forward Primer (see below for the sequence), 0.5 µM Reverse Primer (see below for the sequence), 0.05 mM dDsbnTP, 0.05 mM diol 1-dPxTP (1-(2-deoxy-β-D-ribofuranosyl)-4-[(4-pentyne-1,2-diol)-1-propynyl]-2-nitropyrrole 5'-triphosphate), and 0.05 unit/pL AccuPrime Pfx DNA polymerase. Into the reaction solution having this composition, the single-stranded DNA (T-00198, see below for the sequence) in 4.8 x 10⁹ molecules was added, and PCR products were prepared at 94°C for 2 minutes-(at 94°C for 15 seconds-at 65°C for 210 seconds) x 20 cycles. The PCR products obtained were purified by polyacrylamide gel electrophoresis to isolate single-stranded DNA fragments corresponding to the template DNA. With use of the isolate/purified single-stranded DNA, amplification and purification of DNA fragments were repeated under the PCR conditions described above; thus, a single-stranded DNA was prepared through 100 cycles in total.

**[Table 1]**

| | |
|---|---|
| Forward Primer | TTCTGTCAATCGATCGTATCAGTCCAC |
| Reverse Primer | TTTTTTTTTTTTTTT**6**AAGTAGTCACTAATCCGTTCGAGTCATGC (**6**: C12 linker) |
| T-00198 | TTCTGTCAATCGATCGTATCAGTCCACGGTAAACTGAGTCCGAAG GGGCDsbnTGCAGTGADsbnCCCGAATGGGTCCGGCATGACTCG AACGGATTAGTGACTACTT |

Single-stranded DNAs were prepared in accordance with the above step through 20, 40, or 60 cycles in total, and evaluation was performed by using an LC/MS system (manufactured Waters Corporation, Acquity SQD) to determine whether dDsbn was incorporated in the single-stranded DNAs. A C18 reverse-phase column (Acquity UPLC BEH C₁₈ 1.7 pm, 2.1 x 50 mm) was used for the analysis, and solutions used were mobile phase A (100 mM 1,1,1,3,3,3-hexafluoro-2-propanol, 8 mM triethylamine) and mobile phase B (acetonitrile). Analysis was performed while the mobile phases were flowed at 0.2 mL/min with such a gradient that the percentage of mobile phase B increased from 10% to 50% in 10 minutes. The column temperature at that time was 60°C, and detection was performed at a wavelength of 260 nm (Acquity UPLC PDA). In the analysis, ProMass was used for deconvolution.

First, the single-stranded DNA synthesized by chemical synthesis and used as a template DNA in PCR was analyzed as a control for LC/MS analysis. The results showed that the retention time of the single-stranded DNA was 4.42 seconds, the molecular weight found therefor was 31850.2, and the Mass error from the theoretical value (31852.9) was -2.7 Da (-0.008%) (Table 2). Subsequently, the PCR products were analyzed by LC/MS under the same conditions. The retention time of the main component of the 20-cycle PCR product was 4.45 seconds and the molecular weight found therefor was 31861.8. The retention time of the main component of the 40-cycle PCR product was 4.46 seconds and the molecular weight found therefor was 31859.7. The retention time of the main component of the 60-cycle PCR product was 4.44 seconds and the molecular weight found therefor was 31836.2. Table 2 shows the data. The retention time of the main component contained in each of the DNA products obtained through the 20, 40, and 60 cycles was almost the same as that of the control, and the errors of the molecular weights found from the theoretical molecular weight were 0.05% or smaller. This revealed that dDsbn according to the present embodiment is recognized by DNA polymerase and incorporated in replicated DNA fragments through PCR.

**[Table 2]**

| Sample name (min) | RT | Target Mass (Da) | Observed Mass (Da) | Ma , s Error |
|---|---|---|---|---|
| T-00198 (Synthetic product) | 4.42 | 31852.9 | 31850.2 | -2.7 Da (-0.008%) |
| T-00198 (PCR product 20cycles) | 4.45 | 31852.9 | 31861.8 | 8.9 Da (0.028%) |
| T-00198 (PCR product 40cycles) | 4.46 | 31852.9 | 31859.7 | 6.8 Da (0.021%) |
| T-00198 (PCR product. 60cycles) | 4.44 | 31852.9 | 31836.2 | -16.7 Da (-0.052%) |

### [Example 10]

### Screening using single-stranded DNA library containing nucleoside having artificial base

### <Obtaining DNA aptamer for VEGF165>

A single-stranded DNA library containing dDsbn for use in screening was synthesized with a chemical synthesis method, and the DNAs obtained were purified by polyacrylamide gel electrophoresis.

The single-stranded DNA library with dDsbn contained in random regions was dissolved in PBS solution, and a folding operation (at 95°C for 5 minutes → at room temperature for 20 minutes → on ice for 5 minutes) was performed to form tertiary structures. Thereafter, PBS solution containing NP-40 was added to adjust the solution composition, giving PBS solution containing 0.005% NP-40 (binding solution). The target protein VEGF₁₆₅ was then added, and the resultant was mixed by inversion at room temperature for 30 minutes. To add a biotin tag to the target protein, Ez-Link Sulfo-NHS-Lc-biotin (manufactured by Thermo Fisher Scientific) was added and reacted at room temperature for 15 minutes, and glycine was added to terminate the reaction. Thereafter, solution exchange was performed with an Amicon Ultra Filter cup-0.5 50k (manufactured by Merck KGaA) to remove unreacted reagents.

With use of streptavidin magnetic beads (manufactured by NEW ENGLAND BioLabs, Inc.), the binding single-stranded DNA pool was pulled up by recovering the biotinylated target protein from the sample subjected to solution exchange. The magnetic beads kept in that state were washed with the binding solution to remove single-stranded DNAs with low binding affinity. A single-stranded DNA pool binding to the target protein even after washing was collected from the magnetic beads.

To amplify the single-stranded DNA pool collected, PCR was performed with AccuPrime Pfx DNA polymerase. The composition of PCR reaction solution was as follows: lx AccuPrime Pfx Reaction Mix (containing 0.3 mM dNTP, 1.0 mM MgSO₄), 0.5 mM MgS04, 0.1 mM dNTP, 0.5 µM Forward Primer for selection (see below for the sequence in Table 3), 0.5 µM Reverse Primer for selection (see below for the sequence in Table 3), 0.05 mM dDsbnTP, 0.05 mM diol 1-dPxTP, and 0.05 unit/pL AccuPrime Pfx DNA polymerase. The PCR cycle conditions were at 94°C for 2 minutes-(at 94°C for 15 seconds-at 65°C for 210 seconds) x n cycles. The number of cycles, n, differed among rounds, and cycles were performed enough for amplifying to an amount that allows purification by polyacrylamide gel electrophoresis.

**[Table 3]**

| | |
|---|---|
| Forward Primer for selection | CTCCATTGACTCGGCTTAAACTCCC |
| Reverse Primer for selection | TTTTTTTTTTTTTTT**6**AGCCAAGGAGCACCCCAATATGACC (**6**: C12 linker) |

For purification of a single-stranded DNA pool from amplified DNA fragments, electrophoresis with 10% polyacrylamide gel containing 7 M urea was performed, DNA fragments were extracted from gel pieces containing DNA fragments, and ethanol precipitation was performed for desalting, thereby purifying a single-stranded DNA pool binding to the target protein.

This operation was repeated under varied conditions with different concentrations of the single-stranded DNA pool and the target protein and different washing steps to promote concentration of the sequence binding to the target protein. In each of the second round and the subsequent rounds, to eliminate a single-stranded DNA pool that nonspecifically binds to streptavidin magnetic beads, magnetic beads were added to the pool after the folding operation, and the resultant was mixed by inversion at room temperature for 30 minutes, and the magnetic beads were removed from the pool solution, to which the target protein was then added and reacted.

### <Evaluation of affinity of DNA pool obtained through screening with target protein>

EMSA (electrophoretic mobility shift assay) was performed for the pool containing concentrated single-stranded DNAs that bind to the target protein to determine whether a bindable sequence was contained.

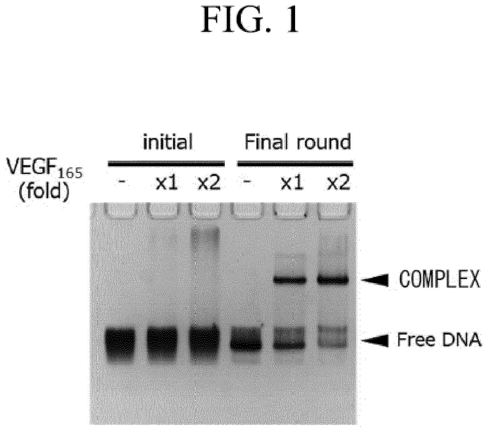

The single-stranded DNA pool was added into the binding solution to adjust to 0.2 µM, and a folding operation (at 95°C for 5 minutes , (-0.1°C/sec) → at 25°C) was performed to form tertiary structures. The target protein was diluted with the binding solution to a predetermined concentration, and mixed with the folded DNA solution, and the resultant was incubated at 37°C for 30 minutes. The sample incubated was subjected to electrophoresis with 10% unmodified polyacrylamide gel. The gel after the electrophoresis was stained with SYBR Gold to detect DNA (Fig. 1). The results showed that clearly higher shift band intensity and thinner Free DNA bands appeared at higher concentration of VEGF165 in the pool in the final round, confirming the presence of a sequence binding to the target protein.

### <Identification of candidate sequence>

Sequences contained in the DNA pool obtained through screening were analyzed by using a next-generation sequencer (Ion PGM system, manufactured by Thermo Fisher Scientific). Clustering of a group of sequences with no variation being found for the length of inserted nucleotides between Forward primer for selection and Reverse Primer for selection and with the artificial base dDsbn remaining therein showed that a single sequence accounted for approximately 80%.

### <Evaluation of binding affinity of candidate sequence with target protein by EMSA>

Analysis was performed by EMSA on whether the candidate sequence identified binds to the target protein.

First, an oligomer corresponding to the candidate sequence was synthesized with a chemical synthesis method, and purified with an OPC (cartridge).

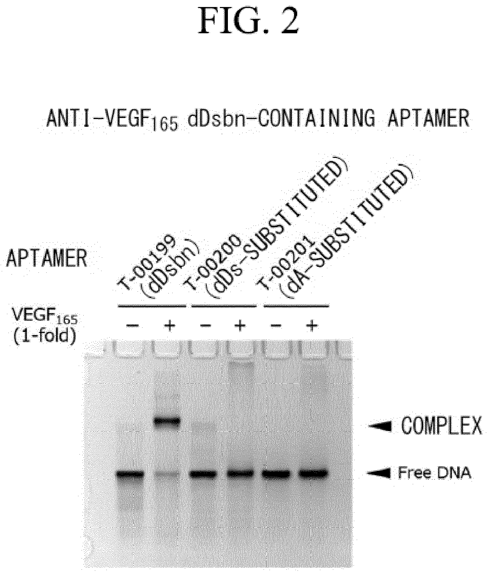

The purified oligomer of the candidate sequence (T-00199, see below for the sequence in Table 4) was added into the binding solution to adjust to 0.2 µM, and a folding operation (at 95°C for 5 minutes , (-0.1°C/sec) → at 25°C) was performed to form tertiary structures. Thereafter, VEGF165 was diluted with the binding solution to 0.2 µM and mixed with the folded DNA solution, and the resultant was incubated at 37°C for 30 minutes. The sample incubated was subjected to electrophoresis with 10% unmodified polyacrylamide gel to analyze on whether a band indicative of the DNA-protein complex could be detected. DNA was stained with SYBR Gold, and detected with blue light at 470 nm. The results showed a new band detected in the presence of VEGF165 (two lanes in the left in Fig. 2); thus, the sequence identified through the present screening was revealed to be an aptamer that binds to VEGF165.

Subsequently, to determine whether the novel artificial base dDsbn is involved in binding to VEGF165, evaluation was performed with oligomers obtained by substituting dDsbn with dDs or dA (dDs-substituted form: T-00200, dA-substituted form: T-00201, see below for the sequences in Table 4). The oligomers were synthesized with a chemical synthesis method, and purified with an OPC (cartridge). For EMSA, a folding operation and reaction were performed as described above, and analysis was performed with unmodified polyacrylamide (in Fig. 2, dDs: two lanes in the center, dA: two lanes in the right). The results showed that substitution of dDsbn with dDs or dA led to the loss of the shift band derived from the complex with the protein. This successfully demonstrated that in the DNA aptamer containing dDsbn, which was identified in the present experiment, dDsbn plays an important role in binding to the target protein VEGF165.

**[Table 4]**

| | |
|---|---|
| T-00199 | CTCCATTGACTCGGCTTAAACTCCCGTCAGTTATGADsbnTAGGTACGCDsbnTGC GGAGTTCGCTACCCCGCCATGGTCATATTGGGGTGCTCCTTGGCT |
| T-00200 | CTCCATTGACTCGGCTTAAACTCCCGTCAGTTATGADsTAGGTACGCDsTGC GGAGTTCGCTACCCCGCCATGGTCATATTGGGGTGCTCCTTGGCT |
| T-00201 | CTCCATTGACTCGGCTTAAACTCCCGTCAGTTATGAATAGGTACGCATGC GGAGTTCGCTACCCCGCCATGGTCATATTGGGGTGCTCCTTGGCT |

### <Obtaining DNA aptamer for sIL-6R>

Concentration of a DNA sequence that binds to sIL-6R was performed by using the same DNA library containing Dsbn for VEGF165 as described above in the same procedure for screening.

### <Evaluation of affinity of DNA pool obtained through screening with target protein>

Evaluation was performed by EMSA on whether a DNA sequence that binds to sIL-6R was contained in the DNA pool obtained by progressing screening.

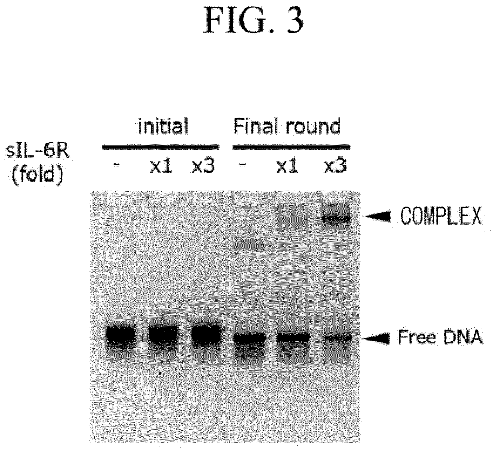

A folding operation to form three-dimensional structures was performed, and the target protein sIL-6R was diluted with the binding solution to a predetermined concentration. For binding reaction, incubation was performed at room temperature for 30 minutes, and the resultant was analyzed with 8% unmodified polyacrylamide gel (Fig. 3). The results showed that thicker shift bands appeared at higher sIL-6R concentration in the DNA pool in the final round, revealing that a DNA sequence binding to sIL-6R was concentrated in the DNA pool.

### <Identification of candidate sequence>

Sequences contained in the DNA pool obtained through screening were analyzed by using a next-generation sequencer (Ion PGM system). Clustering of a group of sequences with no variation being found for the length of inserted nucleotides between Forward primer for selection and Reverse primer for selection and with the artificial base dDsbn remaining therein showed that the most abundant single sequence accounted for approximately 8%.

### <Evaluation of binding affinity of candidate sequence with target protein by EMSA>

Analysis was performed by EMSA on whether the most concentrated candidate sequence binds to the target protein.

First, an oligomer corresponding to the candidate sequence (T-00202, see below for the sequence in Table 5) was synthesized with a chemical synthesis method, and purified with an OPC (cartridge).

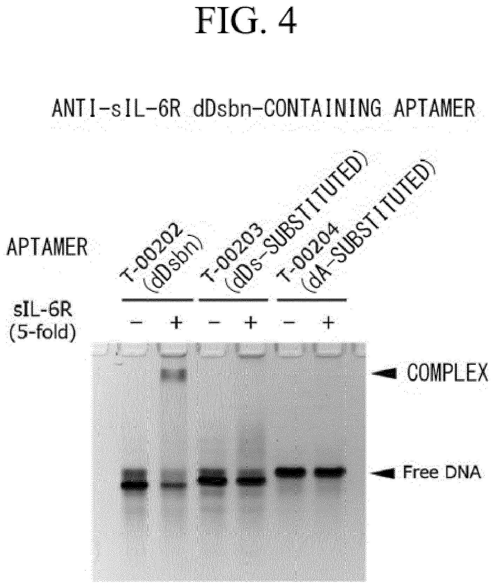

The purified oligomer of the candidate sequence was added into the binding solution to adjust to 0.2 µM, and a folding operation (at 95°C for 5 minutes , (-0.1°C/sec) → at 25°C) was performed to form tertiary structures. Thereafter, sIL-6R was diluted with the binding solution to 0.2 µM and mixed with the folded DNA solution, and the resultant was incubated at room temperature for 30 minutes. The sample incubated was subjected to electrophoresis with 8% unmodified polyacrylamide gel to analyze on whether a band indicative of the DNA-protein complex could be detected. DNA was stained with SYBR Gold, and detected with blue light at 470 nm (two lanes in the left in Fig. 4). The results showed a new band detected in the presence of sIL-6R; thus, the sequence identified through the present screening was revealed to be an aptamer that binds to sIL-6R.

Subsequently, to determine whether the novel artificial base dDsbn is involved in binding to sIL-6R, evaluation was performed with oligomers obtained by substituting dDsbn with dDs or dA (dDs-substituted form: T-00203, dA-substituted form: T-00204, see below for the sequences in Table 5). The oligomers were synthesized with a chemical synthesis method, and purified with an OPC (cartridge). For EMSA, a folding operation, reaction, and analysis with unmodified polyacrylamide were performed as described above (Fig. 4). The results showed that substitution of dDsbn with dDs or dA led to the loss of the shift band derived from the complex with the protein. This successfully demonstrated that in the DNA aptamer containing dDsbn, which was identified in the present experiment, dDsbn plays an important role in binding to the target protein sIL-6R.

**[Table 5]**

| | |
|---|---|
| T-00202 | CTCCATTGACTCGGCTTAAACTCCCAGGAGGTGGTGGGGGTGGGDsbnTTAGG GTTGGGDsbnTTGGAGGGGTGTCGGTCATATTGGGGTGCTCCTTGGCT |
| T-00203 | CTCCATTGACTCGGCTTAAACTCCCAGGAGGTGGTGGGGGTGGGDsTTAGG GTTGGGDsTTGGAGGGGTGTCGGTCATATTGGGGTGCTCCTTGGCT |
| T-00204 | CTCCATTGACTCGGCTTAAACTCCCAGGAGGTGGTGGGGGTGGGATTAGG GTTGGGATTGGAGGGGTGTCGGTCATATTGGGGTGCTCCTTGGCT |

### [Example 11]

### Synthesis of DNA using 3'-amidite of nucleoside having artificial base

In common solid-phase synthesis methods, nucleoside 3'-amidite containing an artificial base according to the present embodiment, an example of which is compound 3, is applicable to synthesis of oligonucleotides under conditions comparable to those for nucleoside 3'-amidite having a natural base. Compound 3 was applied to a solid-phase synthesis method, and the resulting DNA strand (T-00243, see below for the sequence in Table 6) was purified by polyacrylamide gel electrophoresis. Analysis was performed by using a next-generation sequencer (Ion PGM system) on whether dDscp was contained in the DNA strand synthesized.

**[Table 6]**

| | |
|---|---|
| T-00243 | TTCTGTCAATCGATCGTATCAGTCCACGGTAAACTGAGTCCGAAGGGGCDscp TGCAGTGADscpCCCGAATGGGTCCGGCATGACTCGAACGGATTAGTGACTACTT |

### [Example 12]

### Application of nucleoside 5'-triphosphate having artificial base to PCR

To confirm that the nucleotide having the artificial base 7-(5-cyclopentylmethylthiophen-2-yl)-3H-imidazo[4,5-b]pyridin-3-yl (Dscp) as its base moiety (hereinafter, referred to as dDscp) is incorporated in a DNA strand by DNA polymerase and amplified, DNA fragments obtained through PCR were analyzed with a next-generation sequencer.

The single-stranded DNA containing dDscp (T-00243), which was prepared in Example 11, was used as a template DNA for the following PCR reaction.

PCR was carried out with AccuPrime Pfx DNA polymerase. The composition of PCR solution was as follows: lx AccuPrime Pfx Reaction Mix (containing 0.3 mM dNTP, 1.0 mM MgSO₄), 0.5 mM MgSO₄, 0.1 mM dNTP, 0.5 µM Forward Primer, 0.5 µM Reverse Primer, 0.05 mM dDscpTP, 0.05 mM diol 1-dPxTP, and 0.05 unit/pL AccuPrime Pfx DNA polymerase. Into the reaction solution having this composition, the single-stranded DNA (T-00243) in 4.8 x 10⁹ molecules was added, and PCR products were prepared at 94°C for 2 minutes-(at 94°C for 15 seconds-at 65°C for 210 seconds) x 20 cycles. The PCR products obtained were purified by polyacrylamide gel electrophoresis to isolate single-stranded DNA fragments corresponding to the template DNA. With use of the isolate/purified single-stranded DNA, amplification and purification of DNA fragments were repeated under the PCR conditions described above; thus, a single-stranded DNA was prepared through 60 cycles in total.

Evaluation was performed by using a next-generation sequencer (Ion PGM system) to determine whether dDscp was incorporated in the single-stranded DNA prepared through 60 cycles in total in accordance with the aforementioned step. The results confirmed the presence of dDscp at a specific position in the PCR product obtained through 60 cycles in total. This revealed that dDscp was recognized by DNA polymerase and incorporated in replicated DNA fragments.

### [Example 13]

### Screening using single-stranded DNA library containing nucleoside having artificial base, dDscp

### <Obtaining DNA aptamer for VEGF165>

A single-stranded DNA library containing dDscp for use in screening was synthesized with a chemical synthesis method, and the DNAs obtained were purified by polyacrylamide gel electrophoresis. For this single-stranded DNA library, Forward Primer 2 for selection and Reverse Primer 2 for selection (see below for the sequences in Table 7) were used, which are different from the combination of primers used in Example 10.

In a concentration step for a binding sequence to VEGF165 with use of the single-stranded DNA library with dDscp contained in random regions, the same procedure for screening as shown in Example 6 was performed.

**[Table 7]**

| | |
|---|---|
| Forward Primer 2 for selection | GCGTGTTGAGCTGTATTAGGGTGTC |
| Reverse Primer 2 for selection | TTTTTTTTTTTTTTT**6**CCGTCTCATGGATCAGGATTGTATC (**6**: C12 linker) |

### <Evaluation of affinity of DNA pool obtained through screening with target protein>

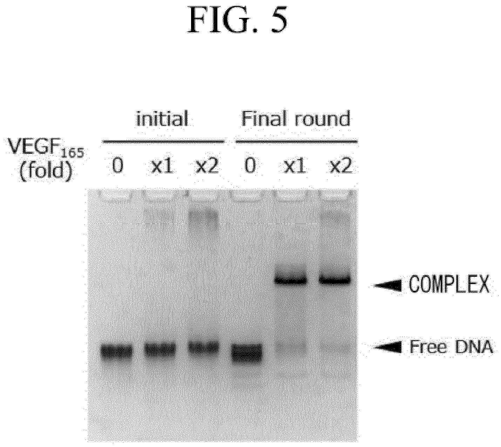

Evaluation was performed by EMSA on whether a DNA sequence that binds to VEGF165 was contained in the DNA pool obtained in the screening step. A folding operation to form three-dimensional structures was performed, and VEGF165 was diluted with the binding solution to a predetermined concentration. The materials were mixed together, and the resultant was incubated at 37°C for 30 minutes, and analyzed by 8% unmodified polyacrylamide gel (Fig. 5). The results showed that clear shift bands indicating binding to VEGF165 were detected in the DNA pool in the final round, revealing that a DNA sequence binding to VEGF165 was concentrated in the DNA pool obtained through screening.

### <Identification of candidate sequence>

Sequences contained in the DNA pool obtained through screening were analyzed by using a next-generation sequencer (Ion PGM system). Clustering of a group of sequences with no variation being found for the length of inserted nucleotides between Forward primer 2 for selection and Reverse Primer 2 for selection and with the artificial base dDscp remaining therein showed that the most abundant single sequence accounted for approximately 30%.

### <Evaluation of binding affinity of candidate sequence with target protein by EMSA>

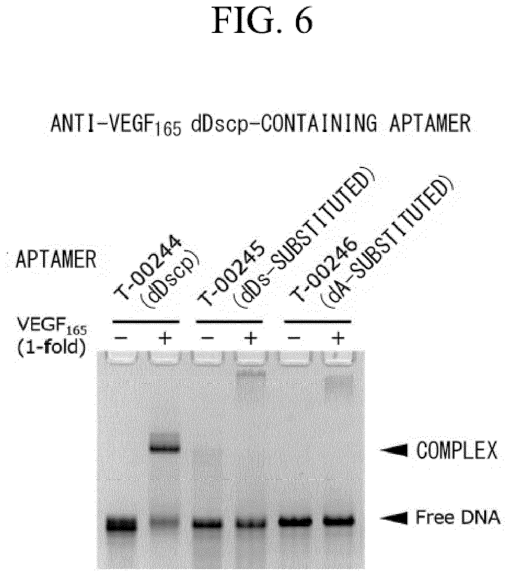

Analysis was performed by EMSA on whether the most concentrated candidate sequence binds to the target protein. First, an oligomer corresponding to the candidate sequence (T-00244, see below for the sequence in Table 8) was synthesized with a chemical synthesis method, and purified with an OPC (cartridge). The purified oligomer of the candidate sequence was added into the binding solution to adjust to 0.2 µM, and a folding operation (at 95°C, for 5 minutes , (-0.1°C/sec) → at 25°C) was performed to form tertiary structures. Thereafter, VEGF165 was diluted with the binding solution to 0.2 µM and mixed with the folded DNA solution, and the resultant was incubated at 37°C for 30 minutes. The sample incubated was subjected to electrophoresis with 8% unmodified polyacrylamide gel to analyze on whether a band indicative of the DNA-protein complex could be detected. DNA was stained with SYBR Gold, and detected with blue light at 470 nm. The results showed a new band detected in the presence of VEGF165 (two lanes in the left in Fig. 6); thus, the sequence identified through the present screening was revealed to be an aptamer that binds to VEGF165.

Subsequently, to determine whether the novel artificial base dDscp is involved in binding to VEGF165, evaluation was performed with oligomers obtained by substituting dDscp with dDs or dA (dDs-substituted form: T-00245, dA-substituted form: T-00246, see below for the sequences in Table 8). The oligomers were synthesized with a chemical synthesis method, and purified with an OPC (cartridge). For EMSA, a folding operation, reaction, and analysis with unmodified polyacrylamide were performed as described above (Fig. 6). The results showed that substitution of dDscp with dDs or dA led to the loss of the shift band derived from the complex with the protein. This successfully demonstrated that in the DNA aptamer identified in the present experiment, dDscp plays an important role in binding to VEGF165.

**[Table 8]**

| | |
|---|---|
| T-00244 | GCGTGTTGAGCTGTATTAGGGTGTCAGGTTTTGGTCAGGCGTTCDscpAACGT AGAGGADscpTGGACACTTCGGCGATACAATCCTGATCCATGAGACGG |
| T-00245 | GCGTGTTGAGCTGTATTAGGGTGTCAGGTTTTGGTCAGGCGTTCDsAACGT AGAGGADsTGGACACTTCGGCGATACAATCCTGATCCATGAGACGG |
| T-00246 | GCGTGTTGAGCTGTATTAGGGTGTCAGGTTTTGGTCAGGCGTTCAAACGT AGAGGAATGGACACTTCGGCGATACAATCCTGATCCATGAGACGG |

### <Obtaining DNA aptamer for sIL-6R>

In a concentration step for a binding sequence to sIL-6R with use of the single-stranded DNA library with dDscp contained in random regions, the same procedure for screening as shown in Example 10 was performed.

### <Evaluation of affinity of DNA pool obtained through screening with target protein>

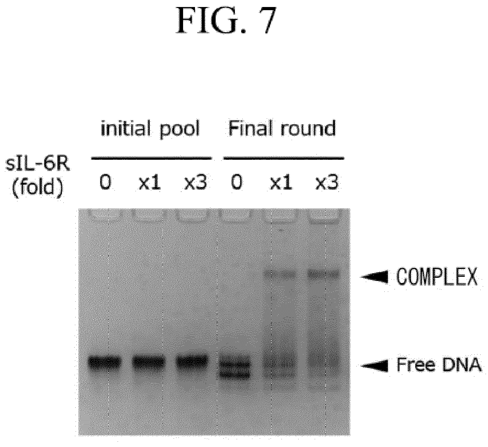

Evaluation was performed by EMSA on whether a DNA sequence that binds to sIL-6R was contained in the DNA pool obtained through the screening step. A folding operation to form three-dimensional structures was performed, and sIL-6R was diluted with the binding solution to a predetermined concentration. The materials were mixed together, and the resultant was incubated at room temperature for 30 minutes, and analyzed with 8% unmodified polyacrylamide gel (Fig. 7). The results showed that clear shift bands indicating binding were detected at higher concentration of sIL-6R in the DNA pool in the final round, revealing that a DNA sequence binding to sIL-6R was concentrated in the DNA pool obtained through screening.

### <Identification of candidate sequence>

Sequences contained in the DNA pool obtained through screening were analyzed by using a next-generation sequencer (Ion PGM system). Clustering of a group of sequences with no variation being found for the length of inserted nucleotides between Forward primer 2 for selection and Reverse Primer 2 for selection and with the artificial base dDscp remaining therein identified a single sequence the abundance of which was as high as approximately 22%.

### <Evaluation of binding affinity of candidate sequence with target protein by EMSA>

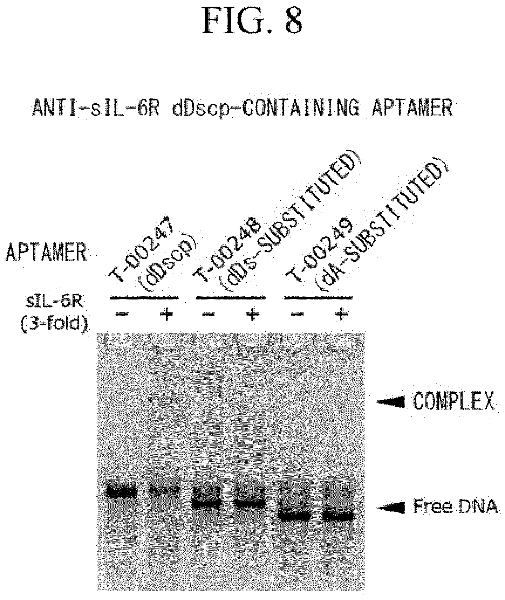

Analysis was performed by EMSA on whether the most concentrated candidate sequence binds to the target protein. First, an oligomer corresponding to the candidate sequence (T-00247, see below for the sequence in Table 9) was synthesized with a chemical synthesis method, and purified with an OPC (cartridge). The purified oligomer of the candidate sequence was added into the binding solution to adjust to 0.2 µM, and a folding operation (at 95°C, for 5 minutes → (-0.1°C/sec) → at 25°C) was performed to form tertiary structures. Thereafter, sIL-6R was diluted with the binding solution to 0.6 µM and mixed with the folded DNA solution, and the resultant was incubated at room temperature for 30 minutes. The sample incubated was subjected to electrophoresis with 8% unmodified polyacrylamide gel to analyze on whether a band indicative of the DNA-protein complex could be detected. DNA was stained with SYBR Gold, and detected with blue light at 470 nm. The results showed a new band detected in the presence of sIL-6R (two lanes in the left in Fig. 8); thus, the sequence identified through the present screening was revealed to be an aptamer that binds to sIL-6R.

Subsequently, to determine whether the novel artificial base dDscp is involved in binding to sIL-6R, evaluation was performed with oligomers obtained by substituting dDscp with dDs or dA (dDs-substituted form: T-00248, dA-substituted form: T-00249, see below for the sequences in Table 9). The oligomers were synthesized with a chemical synthesis method, and purified with an OPC (cartridge). For EMSA, a folding operation, reaction, and analysis with unmodified polyacrylamide were performed as described above (Fig. 8). The results showed that substitution of dDscp with dDs or dA led to the loss of the shift band derived from the complex with the protein. This successfully demonstrated that in the DNA aptamer containing dDscp, which was identified in the present experiment, dDscp plays an important role in binding to sIL-6R.

**[Table 9]**

| | |
|---|---|
| T-00247 | GCGTGTTGAGCTGTATTAGGGTGTCAGCAAGGATTCGGGGGTGGDscpTTTAGG GGGGGDscpTCGTTGTGGAGGCGATACAATCCTGATCCATGAGACGG |
| T-00248 | GCGTGTTGAGCTGTATTAGGGTGTCAGGAAGGATTCGGGGTGGDsTTTAGG GGGGGDsTCGTTGTGGAGGCGATACAATCCTGATCCATGAGACGG |
| T-00249 | GCGTGTTGAGCTGTATTAGGGTGTCAGGAAGGATTCGGGGGTGGATTTAGG GGGGGATCGTTGTGGAGGCGATACAATCCTGATCCATGAGACGG |

The present invention is not limited to the aspects and Examples described above, and includes alternative aspects and modified aspects that can be included in the scope of the present invention specified in claims of the present application.

## Claims

1. A deoxyribonucleoside or deoxyribonucleotide having, as a base, a structure of formula I: 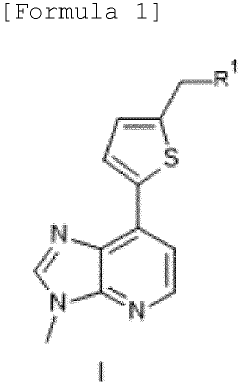 wherein R¹ is a substituted C₁-C₃ alkyl group, a substituted or unsubstituted C₄-C₉ alkyl group, a substituted or unsubstituted aryl group, a substituted or unsubstituted cycloalkyl group, a substituted or unsubstituted aralkyl group, or a substituted or unsubstituted heteroaryl group.

2. The deoxyribonucleoside or deoxyribonucleotide according to claim 1, wherein R¹ is a phenyl group, a 4-pyridyl group, a naphthyl group, an isopropyl group, or a cyclopentyl group.

3. The deoxyribonucleotide according to claim 1 or 2, having triphosphate at the 5'-hydroxy group.

4. The deoxyribonucleoside according to claim 1 or 2, having a protecting group at the 5'-hydroxy group, and having a phosphoramidite group or an H-phosphonate group at the 3'-hydroxy group.

5. A reagent for oligonucleotide synthesis, the reagent comprising the deoxyribonucleoside or deoxyribonucleotide according to any one of claims 1 to 4.

6. A medicament comprising the deoxyribonucleoside or deoxyribonucleotide according to any one of claims 1 to 4.

7. A synthesis method for the deoxyribonucleoside according to claim 1 or 2, the method comprising a step of performing coupling reaction between a 2-bromothiophene derivative and 2-amino-3-nitro-4-chloropyridine or a 7-chloro-3H-imidazo[4,5-b]pyridine derivative.

8. The synthesis method according to claim 7, wherein the coupling reaction is performed under conditions for Suzuki coupling reaction.

9. An oligodeoxyribonucleotide incorporating a deoxyribonucleoside having the base according to claim 1 or 2.

10. An oligonucleotide or a derivative thereof, the oligonucleotide comprising the oligodeoxyribonucleotide according to claim 9 as a substructure.

11. A modified product comprising the oligodeoxyribonucleotide according to claim 9 or the oligonucleotide or derivative thereof according to claim 10 bound to any independent molecular species.

12. A medicament for a human or an animal, a raw material of the medicament, an additive for a health food, or a diagnostic reagent, wherein the medicament, the raw material, the additive, or the diagnostic reagent comprises the oligodeoxyribonucleotide according to claim 9, the oligonucleotide or derivative thereof according to claim 10, or the modified product according to claim 11.

13. A medical article comprising the oligodeoxyribonucleotide according to claim 9, the oligonucleotide or derivative thereof according to claim 10, or the modified product according to claim 11.

14. The medical article according to claim 13, which is a medical ingredient, a medical material, a medical device, or a medical product.

15. A carrier for use in affinity column chromatography, the carrier comprising the oligodeoxyribonucleotide according to claim 9, the oligonucleotide or derivative thereof according to claim 10, or the modified product according to claim 11.

16. A column, a substance separator, or a medical device, wherein the column, the substance separator, or the column comprises the carrier according to claim 15.
